# EUROPÄISCHE PATENTSCHRIFT

(11) **EP 1 805 328 B1**
(45) Veröffentlichungstag und Bekanntmachung des Hinweises auf die Patenterteilung: **14.01.2009**
(21) Anmeldenummer: 05803571.8
(22) Anmeldetag: 08.10.2005
(51) Int. Cl.: C14C 3/20

(54) **VERFAHREN ZUR HERSTELLUNG VON LEDER UND DAFÜR GEEIGNETE VERBINDUNGEN**
PROCESS FOR PRODUCING LEATHER AND COMPOUNDS SUITABLE THEREFOR
PROCEDE DE PRODUCTION DE CUIR ET COMPOSES APPROPRIES

(30) Priorität: 18.10.2004 DE 102004050879
(43) Veröffentlichungstag der Anmeldung: 11.07.2007
(73) Patentinhaber: BASF SE, 67056 Ludwigshafen (DE)
(72) Erfinder: RÜBA, Eva, 68161 Mannheim (DE); ERHARDT, Rainer, 68199 Mannheim (DE); REESE, Oliver, 67061 Ludwigshafen am Rhein (DE); SOMOGYI, Laszlo, 67117 Limburgerhof (DE); ZAMPONI, Andrea, 68167 Mannheim (DE); KIESOW, Harald, 67067 Ludwigshafen (DE); PABST, Gunther, 92318 Neumarkt (DE); HÜFFER, Stephan, 67063 Ludwigshafen (DE); DANISCH, Peter, 67065 Ludwigshafen (DE); SCHROEDER, Stefan, 67271 Neuleiningen (DE)
(86) Internationale Anmeldenummer: PCT/EP2005/010855
(87) Internationale Veröffentlichungsnummer: WO 2006/042652

(56) Entgegenhaltungen:
- EP-A- 0 337 063
- WO-A-01/55456
- WO-A-01/68584
- DE-A1- 2 750 990
- DE-A1- 19 713 112

## Beschreibung

Die vorliegende Erfindung betrifft ein Verfahren zur Herstellung von Leder, dadurch gekennzeichnet, dass man vorbehandelte Tierhäute mit wenigstens einer Verbindung kontaktiert, die wenigstens eine Gruppe der Formel I aufweist, 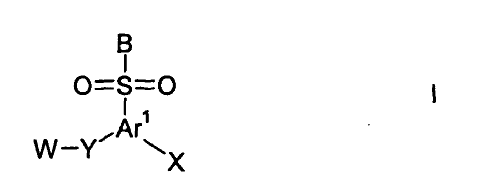 wobei die Variablen wie folgt definiert sind:
- Ar¹: ein aromatisches oder heteroaromatisches System,
- X: ein elektronenziehender Rest oder Wasserstoff,
- Y: eine bivalente funktionelle Gruppe oder eine Einfachbindung oder eine C₂-C₂₀-Alkylengruppe, bei der bis zu 3 nicht-benachbarte CH₂-Gruppen durch Sauerstoff oder -NR³- ersetzt sein können,
- W: ein Rest, der von einer Verbindung abgeleitet ist, die auf Leder hydrophobierend, fettend oder gerbend wirkt,
- B: gewählt aus -C(R¹)=CH₂ und CHR¹-CH₂-Q, wobei
- R¹: gewählt wird aus Wasserstoff, C₁-C₁₀-Alkyl und Phenyl
- R³: gleich oder verschieden, aus Phenyl, Wasserstoff oder C₁-C₁₀-Alkyl und
- Q: aus unter wässrig-alkalischen Bedingungen abspaltbaren Gruppen.

Weiterhin betrifft die vorliegende Erfindung Leder, hergestellt nach dem erfindungsgemäßen Verfahren. Weiterhin betrifft die vorliegende Erfindung Verbindungen, enthaltend wenigstens eine Gruppe der allgemeinen Formel I, und wässrige Formulierungen von Verbindungen, die wenigstens eine Verbindung der allgemeinen Formel I enthalten.

Gerben, Hydrophobieren und Fetten sind wichtige Schritte bei der Herstellung von Leder. Durch das Gerben werden beispielsweise zahlreiche Gebrauchseigenschaften von Leder entscheidend beeinflusst. Durch das Hydrophobieren und das Fetten werden die haptischen Eigenschaften und das Verhalten gegenüber Nässe und Feuchtigkeit entscheidend beeinflusst. An moderne Gerb-, Hydrophobier- und Fettungsmittel werden daher anspruchsvolle Anforderungen gestellt. Sie sollen möglichst gut aus wässriger Flotte aufzutragen sein, sich gleichmäßig über das zu fettende Leder verteilen, eine gute Flottenauszehrung aufweisen, d.h. mit guter Ausbeute aufgetragen werden, und zur Herstellung von Ledern führen, die höchsten Ansprüchen genügen, beispielsweise Schuhleder, Möbelleder und Autoleder. Auch bei längerem Gebrauch unter klimatisch anspruchsvollen Bedingungen sollen Leder nicht brüchig werden, Fettungsmittel nicht ausschwitzen und keine Flecken bilden. Außerdem muss die Schweißechtheit von Leder, insbesondere Automobilleder und Schuhoberleder, gut sein. Weiterhin ist in verschiedenen Fällen eine hohe Waschechtheit erwünscht.

Aus WO 01/55456 sind Cyanharnstoff-haltige Polysiloxane bekannt, die zur Fettung von Leder eingesetzt werden. Die genannten Verbindungen weisen jedoch beim Gebrauch als Fettungsmittel noch Nachteile auf.

Aus DE 1 078 580 sind sulfochlorierte Licker und ihre Verwendung bei der Herstellung von vegetabil gegerbtem Leder bekannt. Nachteilig bei der Verwendung von sulfochlorierten Lickem ist jedoch, dass man Chlorid-haltige Abwässer erhält.

Es bestand daher die Aufgabe, ein neues Verfahren zur Herstellung von Leder bereit zu stellen, das die Nachteile, die mit den aus dem Stand der Technik verbunden sind, vermeidet. Weiterhin bestand die Aufgabe, neue Leder und ihre Verwendung bereit zu stellen. Weiterhin bestand die Aufgabe, neue Verbindungen bereit zu stellen, unter deren Verwendung sich neue Leder herstellen lassen.

Demgemäß wurden das eingangs definierte Verfahren gefunden.

Das erfindungsgemäße Verfahren geht aus von nach konventionellen Methoden vorbehandelten Tierhäuten, den sogenannten Blößen, von beispielsweise Rindern, Schweinen, Ziegen oder Hirschen. Dabei ist es für das erfindungsgemäße Verfahren nicht wesentlich, ob die Tiere beispielsweise durch Schlachten getötet wurden oder an natürlichen Ursachen verendet sind. Zu den konventionellen Methoden der Vorbehandlung gehören das beispielsweise das Äschern, Entkälken, Beizen und Pickeln sowie mechanische Arbeitsschritte, beispielsweise das Entfleischen der Häute.

Erfindungsgemäß kontaktiert man vorbehandelte Tierhäute mit wenigstens einer Verbindung, die wenigstens eine Gruppe der Formel aufweiset, 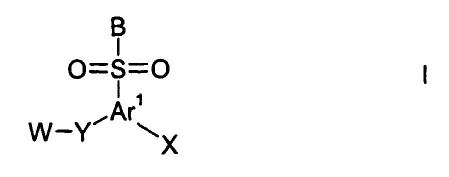 wobei die Variablen wie folgt definiert sind:
- Ar¹: ist ein aromatisches oder heteroaromatisches System, das neben den Resten Y-W, SO₂-B und X weitere Substituenten tragen kann, beispielsweise einen oder mehrere C₁-C₁₀-Alkylreste oder auch weitere elektronenziehende Reste, wenn die Zahl der freien Valenzen an Ar¹ dies zulässt. Bevorzugt wird A¹ gewählt aus Benzolsystemen, Naphthalinsystemen, Anthracensystemen, Phenanthrensystemen, Pyridinsystemen, Chinolinsystemen, Isochinolinsystemen, Acridinsystemen, Pyrazinsystemen, Pyridazinsystemen, Pyrimidinsystemen, Chinazolinsystemen, Chinooxalinsystemen und Triazinsystemen. Ganz besonders bevorzugt sind Benzolsysteme, Naphthalinsysteme und 1,3,5-Triazinsysteme.
- X: ist Wasserstoff oder ein elektronenziehender Rest, d.h. ein Rest, der einen -I-Effekt oder einen -M-Effekt oder beides zeigt, bevorzugt gewählt aus NO₂-Gruppen, CF₃-Gruppen und ganz besonders bevorzugt aus SO₃M-Gruppen.
- Y: ist eine bivalente funktionelle Gruppe oder eine Einfachbindung oder eine C₂-C₂₀-Alkylengruppe, bei der bis zu 3 nicht-benachbarte CH₂-Gruppen durch Sauerstoff oder -NR³- ersetzt sein können. Beispiele für bivalente funktionelle Gruppen sind -O-, -S-. Bevorzugte bivalente funktionelle Gruppen sind die Gruppen Y.1 bis Y.9 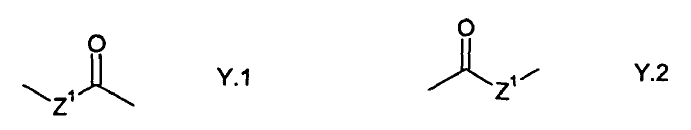 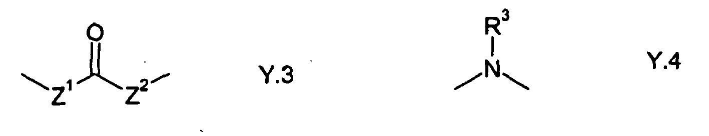 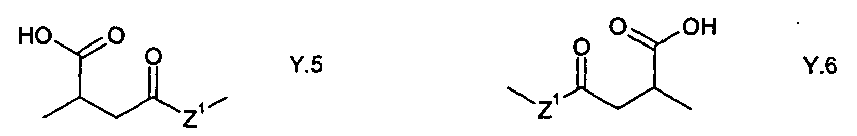 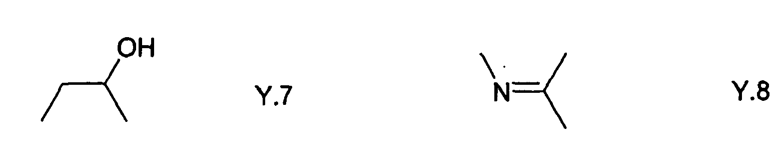 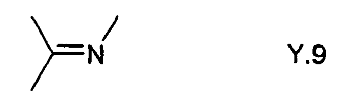
wobei
Z¹ und Z² gleich oder verschieden sein können und gewählt aus Sauerstoff und N-R³.

Eine andere bevorzugte bivalente funktionelle Gruppe Y ist die Diazogruppe -N=N- für den Fall, dass X Wasserstoff bedeutet.

Beispiele für C₂-C₂₀-Alkylengruppen, in denen bis zu 3 nicht-benachbarte CH₂-Gruppen durch NR³ oder Sauerstoff ersetzt sein können, sind -CH₂-CH₂-, -C(CH₃)₂-, -CH₂-CH(CH₃)-, -(CH₂)₃-, -CH₂-CH(C₂H₅)-, -(CH₂)₄-, -(CH₂)₅-, -(CH₂)₆-, -(CH₂)₇-, -(CH₂)₈-, -(CH₂)₉-, -(CH₂)₁₀-; -(CH₂)₂₀-, -CH₂-, -CH₂-CH₂-, -C(CH₃)₂-, -CH₂-CH(CH[CH₃]₂) -CH₂-CH(n-C₃H₇)-, -[CH(CH₃)]₂-, -CH(CH₃)CH₂-CH₂-CH(CH₃)-, -CH(CH₃)-CH₂-CH(CH₃)-, -CH2-C(CH₃)₂-CH₂-, -CH₂-CH(n-C₄H₉)-, -CH₂-CH(t-C₄H₉)-. -CH₂-CH(C₂H₅)-, -CH₂-O-, -CH₂-O-CH₂-, -CH₂-NH-. -CH₂-N(CH₃)-, -(CH₂)₃-NH-(CH₂)₂-NH-, -(CH₂)₂-O-(CH₂)₂-, -[(CH₂)₂-O]₂-(CH₂)₂-, -[(CH₂)₂-O]₃-(CH₂)₂-.
- B: gewählt aus -C(R¹)=CH₂ und CHR¹-CH₂-Q, wobei
- R¹: gewählt wird aus Phenyl,
C₁-C₁₀-Alkyl, verzweigt oder unverzweigt, wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl und ganz besonders bevorzugt Methyl, und insbesondere Wasserstoff.
- R³: sind gleich oder verschieden und unabhängig voneinander gewählt aus Was- , serstoff, Phenyl oder
C₁-C₁₀-Alkyl, verzweigt oder unverzweigt, wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl und ganz besonders bevorzugt Methyl.
- Q: steht für unter wässrig-alkalischen Bedingungen abspaltbaren Gruppen, beispielsweise Alkoxylatgruppen, Halogenid, bevorzugt sind für O-SO₃M, OSO₂-Ar² oder O-CO-R² steht.
- M: steht für Wasserstoff
oder Alkalimetall wie Li, Na, K, Rb oder Cs, bevorzugt Na oder K, oder Ammonium, ausgewählt aus NH₄, C₁-C₁₀-Alkylammonium, Di-C₁-C₁₀-alkyl-ammonium, Tri-C₁-C₁₀-alkylammonium, Tetra-C₁-C₁₀-alkylammonium, wobei C₁-C₁₀-Alkyl gleich oder verschieden sein können und wie oben stehend definiert, Arylammonium wie beispielsweise Anilinium, Aryldialkylammonium wie beispielsweise Dimethylphenylammonium, ω-Hydroxyalkylammonium wie beispielsweise 2-Hydroxyethylammonium, 3-Hydroxypropylammonium, Di-2-hydroxyalkylammonium, Alkyl-ω-Hydroxyalkylammonium wie beispielsweise N-Methyl-2-Hydroxyethylammonium, N,N-Dimethyl-2-hydroxyethylammonium, N-n-Butyl-2-Hydroxyethylammonium.
- Ar²: steht für Phenyl, unsubstituiert oder ein- oder mehrfach substituiert mit Halogen, NO₂, C₁-C₁₀-Alkyl, das wie oben definiert ist, oder SO₃M. Bevorzugt steht Ar² für Phenyl, para-Toluyl und para-Nitrobenzyl.
- R²: steht für Wasserstoff, Phenyl oder C₁-C₁₀-Alkyl, verzweigt oder unverzweigt, wie beispielsweise Methyl, Ethyl, n-Propyl, iso-Propyl, n-Butyl, iso-Butyl, sec.-Butyl, tert.-Butyl, n-Pentyl, isoPentyl, sec.-Pentyl, neo-Pentyl, 1,2-Dimethylpropyl, iso-Amyl, n-Hexyl, isoHexyl, sec.-Hexyl, n-Heptyl, n-Octyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl und ganz besonders bevorzugt Methyl.
- W: ist ein Rest, der von einer Verbindung abgeleitet ist, die auf Leder hydrophobierend, fettend oder gerbend wirkt. Darunter sind solche Gruppen zu verstehen, die - mit Wasserstoff abgesättigt - zu Verbindungen führen, die auf Leder hydrophobierend, fettend oder gerbend wirken. Beispiele für bevorzugte hydrophobierend und/oder fettend wirkende Gruppen sind mono-, di- und poly-funktionale Polysiloxanreste, C₈-C₅₀₀-Alkylreste, verzweigt oder unverzweigt, beispielsweise Polyisobutylenreste mit 2 bis 125, bevorzugt 5 bis 50 Isobutyleneinheiten, C₈-C₆₀-Alkyleneinheiten, verzweigt oder unverzweigt, mehrfach fluorierte C₁-C₂₀-Alkylreste, beispielsweise CF₃, n-C₄F₉, CH₂-CH₂-CF₃, -CH₂-CH₂-C₂F₅, CH₂-CH₂-n-C₃F₇, CH₂-CH₂-iso-C₃F₇, CH₂-CH₂-n-C₄F₉, CH₂-CH₂-n-C₆F₁₃, CH₂-CH₂-n-C₈F₁₇, CH₂-CH₂-n-C₁₀F₂₁, CH₂-CH₂-n-C₁₂F₂₅,
Copolymerisate von Maleinsäureanhydrid und C₁₀-C₃₀-α-Olefinen, Copolymerisate von Maleinsäureanhydrid und Polyisobuten mit 2 bis 125, bevorzugt 5 bis 50 Isobutyleneinheiten.

Beispiele für gerbend wirkende Gruppen sind
Polyacrylsäuren,
Polymethacrylsäuren,
Phenolsulfonsäure-Harnstoff-Formaldehyd-Kondensate, vorzugsweise mit einem Molekulargewicht Mₙ im Bereich von 300 bis 100.000 g/mol, Naphthalinsulfonsäure-Dihydroxydiphenylsulfon-Formaldehyd-Kondensate,
Phenolsulfonsäure-Dihydroxydiphenylsulfon-Hamstoff-Formaldehyd-Kondensate,
Phenolsulfonsäure-Formaldehyd-Kondensate,
Naphthalinmono- und Naphthalindisulfonsäure-Formaldehyd-Kondensate,
Naphthalinmono- und Naphthalindisulfonsäure-Hamstoff-Formaldehyd-Kondensate,
Melamin-Harnstoff-Formaldehyd-Kondensate,
Melamin-Formaldehyd-Kondensate,
Bisphenol-A-Formaldehyd-Kondensate,
Bisphenol-A-Hamstoff-Formaldehyd-Kondensate.

Beispiele für monofunktionale Polysiloxanreste lassen sich beispielsweise durch die Formeln W1.1 bis W.1.3 wiedergeben: 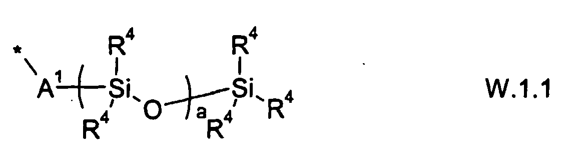 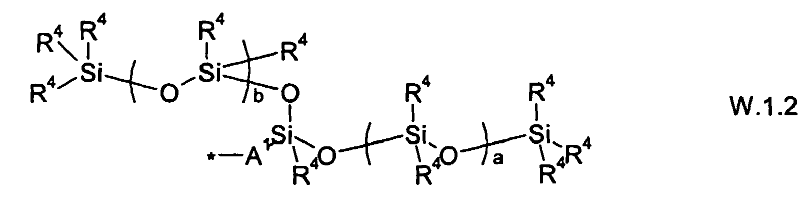 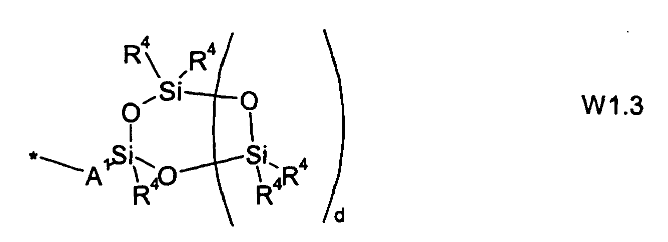 wobei die Variablen wie folgt definiert sind:
- R⁴: gleich oder verschieden und unabhängig voneinander gewählt aus Phenyl und C₁-C₁₀-Alkyl, verzweigt oder unverzweigt, wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl und ganz besonders bevorzugt Methyl, 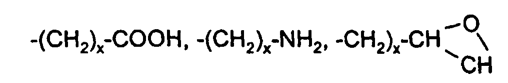
- x: eine ganze Zahl im Bereich von 1 bis 20, bevorzugt 5 bis 14,
- A¹: gleich oder verschieden und unabhängig voneinander eine Einfachbindung oder C₁-C₂₀-Alkylen, verzweigt oder unverzweigt, beispielsweise -CH₂, -(CH₂)₂-, -CH(CH₃)-, -C(CH₃)₂-, -CH₂-CH(CH₃)-, -(CH₂)₃-, -CH₂-CH(C₂H₅)-, -(CH₂)₄-, -(CH₂)₅-, -(CH₂)₆-, -(CH₂)₇-, -(CH₂)₈-, -(CH₂)₉-, -(CH₂)₁₀ -(CH₂)₂₀-;

Dabei können in A¹ bis zu drei nicht-benachbarte CH₂-Gruppen durch Sauerstoff oder NR³-Gruppen ersetzt sein, die jeweils gleich oder verschieden sein können.
- a: steht für eine ganze Zahl im Bereich von 1 bis 80, bevorzugt 3 bis 50 und besonders bevorzugt 10 bis 30,
- b: steht für eine ganze Zahl im Bereich von 0 bis 80, bevorzugt 1 bis 50 und besonders bevorzugt bis 30,
- d: steht für eine ganze Zahl im Bereich von 1 bis 3.

Beispiele für bifunktionelle Polysiloxanreste lassen sich beispielsweise durch die Formeln W2.1.1 bis W2.1.3 wiedergeben: 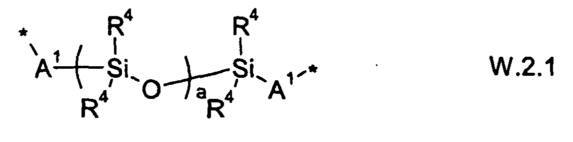 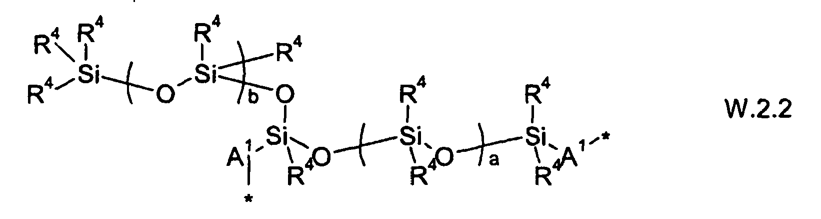 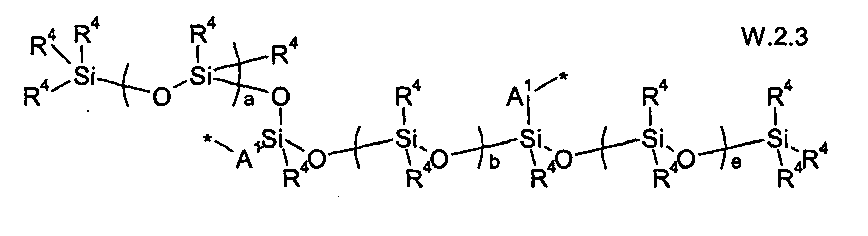 wobei
- e: eine ganze Zahl im Bereich von 0 bis 80, bevorzugt 1 bis 50 und besonders bevorzugt 10 bis 30 bedeutet.

Beispiele für polyfunktionale Polysiloxanreste sind 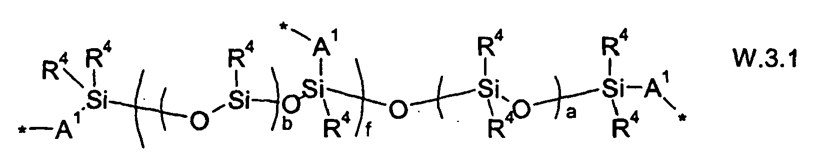 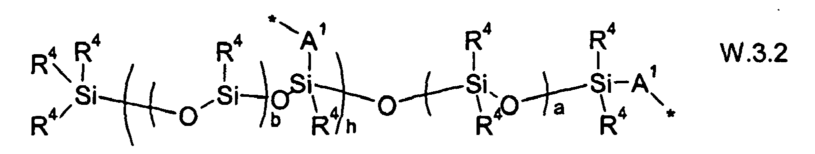 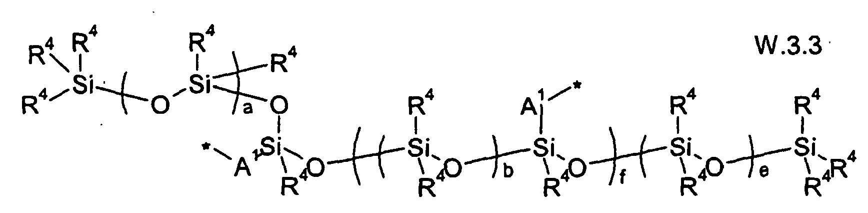 wobei
- f: eine ganze Zahl im Bereich von 1 bis 10 ist, bevorzugt 2 bis 5,
- h: eine ganze Zahl im Bereich von 1 bis 10 ist, bevorzugt 2 bis 5,
- und b: jeweils gleich oder verschieden sein kann und wie oben definiert ist.

Beispiele für C₈-C₆₀-Alkylreste sind n-Octyl, n-Decyl, n-Dodecyl, iso-Dodecyl, n-Tetradecyl, n-Hexadecyl, n-Octadecyl, n-Eicosyl, n-C₃₀H₆₁, n-C₄₀H₈₁, n-C₅₀H₁₀₁, n-C₆₀H₁₂₁, weiterhin Polyisobutylenreste mit 2 bis 50 Isobutyleneinheiten, bevorzugt 5 bis 20.

Beispiele für C₈-C₆₀-Alkyleneinheiten, verzweigt oder unverzweigt, sind -(CH₂)₈-, -(CH₂)₁₀-, -(CH₂)₁₂. -(CH₂)₁₄-, -(CH₂)₁₆-, -(CH₂)₂₀-, -(CH₂)₃₀-, -(CH₂)₄₀-_{,} -(CH₂)₅₀-, -(CH₂)₆₀-, -CH(CH₃)-(CH₂)₆-CH(CH₃)-, -CH(CH₃)-(CH₂)₈-CH(CH₃)-, -CH(C₂H₅)-(CH₂)₆-CH(C₂H₅)-.

Zur Durchführung des erfindungsgemäßen Verfahrens kann man beispielsweise so vorgehen, dass man eine oder mehrere vorbehandelte Tierhäute nach der Vorgerbung, nach der Gerbung, nach der Nachgerbung, bevorzugt vor der Nachgerbung mit mindestens einer Verbindung behandelt, die wenigstens eine Gruppe der Formel I aufweist.

Wenn in der Gruppe der Formel I B gleich -C(R¹)=CH₂ gewählt wird, so kann man bei pH-Werten im Bereich von 3 bis 11 arbeiten. Wenn in der Gruppe der Formel I B gleich -C(R¹)=CH₂ oder -CHR¹-CH₂-Q gewählt wird, so ist es bevorzugt, bei pH-Werten im Bereich von 7 bis 11 zu arbeiten. Besonders bevorzugt arbeitet man bei pH-Werten im Bereich von 8 bis 10.

Um den für eine Fixierung bevorzugten alkalischen pH-Wert einzustellen, kann man beliebige Alkalien und/oder Puffersysteme einsetzen. Beispielhaft zu nennen sind Alkalimetallcarbonate und Alkalimetallhydrogencarbonate wie beispielsweise Natriumcarbonat, Kaliumcarbonat und Natriumhydrogencarbonat, weiterhin Alkalimetallhydroxide wie beispielsweise Natriumhydroxid und Kaliumhydroxid, weiterhin Natriummetasilikat, Pyrophosphate wie beispielsweise Natriumpyrophosphat und Kaliumpyrophosphat, Trialkaliumphosphat wie beispielsweise Trinatriumphosphat, Borax-Natronlauge-Puffer und Phosphatpuffer.

Wünscht man das erfindungsgemäße Verfahren nach der Vorgerbung, nach der Gerbung bzw. nach der Nachgerbung durchzuführen, so ist es an sich unkritisch, ob man die jeweilige Vorgerbung, Gerbung bzw. Nachgerbung mit Chromgerbstoffen, Mineralgerbstoffen, Polymergerbstoffen, Aldehydgerbstoffen wie beispielsweise Glutardialdehyd oder mit vegetabilen Gerbstoffen durchgeführt hat.

In einer Ausführungsform des erfindungsgemäßen Herstellverfahrens gibt man mindestens eine Verbindung mit mindestens einer Gruppe der allgemeinen Formel I in einer oder mehreren Portionen zu der zu behandelnden vorbehandelten Tierhaut. Diese Zugabe kann in einer wässrigen Flotte geschehen. Vorzugsweise kann die Flottenlänge 50 bis 2000 Gew.-%, bevorzugt 100 bis 400 Gew.% betragen, bezogen auf das Falzgewicht der gegerbten Tierhaut bzw. das Nassgewicht des Fells.

Das erfindungsgemäße Verfahren führt man im Allgemeinen so durch, dass man die zu behandelnde Tierhaut in geeigneten Gefäßen walkt, beispielsweise in Fässern, insbesondere in drehbaren Fässern mit Einbauten.

Als Temperatur für das erfindungsgemäße Verfahren kann man Temperaturen im Bereich von 10 bis 65°C, bevorzugt 30 bis 60°C wählen.

Die Druckbedingungen des erfindungsgemäßen Verfahrens sind im Allgemeinen unkritisch. Bevorzugt arbeitet man bei Normaldruck (1 atm), man kann aber auch bei verringertem Druck wie beispielsweise 0,5 bis 0,99 atm oder bei erhöhtem Druck wie beispielsweise 1,01 bis 2 atm arbeiten.

Man beendet das erfindungsgemäße Verfahren im Allgemeinen nach einer Zeit von 20 Minuten bis 24 Stunden, bevorzugt 30 Minuten bis 12 Stunden, besonders bevorzugt bis 4 Stunden. Wenn man die Behandlung wiederholt durchführt, so spricht man im Rahmen der vorliegenden Erfindung von erfindungsgemäßen Behandlungsschritten.

Während der Durchführung des erfindungsgemäßen Verfahrens kann man der Flotte übliche Lederfarbstoffe zusetzen. Beispielhaft seien saure, substantive oder basische Anilinfarbstoffe genannt, die man in gerbereiüblichen Mengen einsetzen kann.

In einer speziellen Ausführungsform der vorliegenden Erfindung setzt man der Flotte solche Farbstoffe zu, die analog zu erfindungsgemäßen Verbindungen der allgemeinen Formel I aufgebaut sind, jedoch statt der Gruppe W eine Gruppe W* aufweisen, wobei W* für einen Chromophor steht.

Bevorzugte Chromophore sind solche aus der Phthalocyaninreihe, weiterhin Anthrachinone, Azofarbstoffe, Formazanfarbstoffe, Triphenyldioxazinfarbstoffe und Triphenylmethanfarbstoffe.

Derartige Chromophore sind aus dem Stand der Technik bekannt, siehe beispielsweise WO 94/18381, EP-A 0 356 931, EP-A 0 559 617, EP-A 0 201 868, DE-A 195 23 245, DE-A 197 31 166, EP-A 0 745 640, EP-A 0 880 098 oder können in Analogie zu strukturell ähnlichen Chromophore hergestellt werden, wie sie beispielsweise aus EP-A 0 602 562, EP-A 0 597 411, EP-A 0 592 105 und DE 43 19 674 bekannt sind.

In einer speziellen Ausführungsform der vorliegenden Erfindung führt man das erfindungsgemäße Verfahren als oder mit der Nachgerbung durch.

Wünscht man das erfindungsgemäße Verfahren in einem Schritt mit der Nachgerbung durchzuführen, so kann man mit beliebigen in der Gerberei üblichen Gerbstoffen, beispielsweise Mineralgerbstoffen, insbesondere Chromgerbstoffen, oder Polymergerbstoffen, Syntanen, Harzgerbstoffen, vegetabilen Gerbstoffen oder Kombinationen aus den vorgenannten Gerbstoffen arbeiten.

In einer speziellen Variante der vorliegenden Erfindung, in der W aus Formel I von einer Verbindung abgeleitet ist, die auf Leder gerbend wirkt, führt man das erfindungsgemäße Verfahren als Nachgerbung aus und verzichtet auf die Zugabe von weiteren in der Gerberei üblichen Gerbstoffen.

In einer speziellen Variante der vorliegenden Erfindung, in der W aus Formel I von einer Verbindung abgeleitet ist, die auf Leder gerbend wirkt, führt man das erfindungsgemäße Verfahren als Nachgerbung unter Zugabe von an sich bekannten Fettungsmitteln durch, beispielsweise Polydimethylsiloxan, natürlichen oder synthetischen Wachsen, nativen oder synthetischen Ölen oder anderen aus Hersfeld, Bibliothek des Leder, Band 4, "Entfetten, Fetten und Hydrophobieren bei der Lederherstellung", 1987 bekannten Fettungsmitteln aus.

In einer anderen speziellen Variante, in der W aus Formel I von einer Verbindung abgeleitet ist, die hydrophobierend oder fettend wirkt, führt man das erfindungsgemäße Verfahren unter Zugabe von an sich bekannten Fettungsmitteln durch, beispielsweise Polydimethylsiloxan, natürliche oder synthetische Wachse, native oder synthetische Öle, oder anderen aus Hersfeld, Bibliothek des Leder, Band 4, "Entfetten, Fetten und Hydrophobieren bei der Lederherstellung" 1987, bekannten Fettungsmitteln aus.

In einer anderen speziellen Variante, in der W aus Formel I von einer Verbindung abgeleitet ist, die auf Leder hydrophobierend oder fettend wirkt, verzichtet man auf die Zugabe von weiteren in der Gerberei üblichen Hydrophobier- und Fettungsmitteln.

In einer speziellen Variante der vorliegenden Erfindung arbeitet man mit mindestens zwei verschiedenen erfindungsgemäßen Verbindungen, und zwar mit mindestens einer erfindungsgemäßen Verbindung, die wenigstens eine Gruppe der Formel I aufweist, die auf Leder gerbend wirkt, und mit mindestens einer weiteren erfindungsgemäßen Verbindung, die wenigstens eine Gruppe der Formel I aufweist, die auf Leder fettend und/oder hydrophobierend wirkt.

Ein weiterer Gegenstand der vorliegenden Erfindung sind Leder, hergestellt nach dem erfindungsgemäßen Herstellverfahren. Nach dem erfindungsgemäßen Verfahren hergestellte Leder zeichnen sich durch sehr gute Gebrauchseigenschaften aus, beispielsweise durch sehr gute Hydrophobie, sehr guten Griff, hervorragend egale Färbung und sehr gute Waschbarkeit, weiterhin sehr gute Fülle und Weichheit.

Ein weiterer Gegenstand der vorliegenden Erfindung ist die Verwendung der erfindungsgemäßen Leder zur Herstellung von Bekleidungsstücken, beispielsweise Jacken, Mänteln, Schuhen und insbesondere Stiefeln. Ein weiterer Gegenstand der vorliegenden Erfindung ist die Verwendung der erfindungsgemäßen Leder zur Herstellung von Möbeln und Möbelteilen, beispielsweise Ledersofas, Ledersesseln, Armlehnen für Stühle, Sessel oder Sofas oder Bänke. Ein weiterer Gegenstand der vorliegenden Erfindung ist die Verwendung der erfindungsgemäßen Leder zur Herstellung von Autoteilen, beispielsweise Autositzen, Teilen von Armaturenbrettern und Innenverkleidungsteilen, beispielsweise in Autotüren.

Ein weiterer Gegenstand der vorliegenden Erfindung sind Verbindungen, enthaltend mindestens eine Gruppe der allgemeinen Formel I 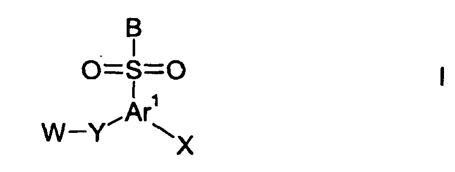 wobei die Variablen wie folgt definiert sind:
- Ar¹: ist ein aromatisches oder heteroaromatisches System, das neben den Resten Y-W, SO₂-B und X weitere Substituenten tragen kann, beispielsweise einen oder mehrere C₁-C₁₀-Akylreste oder auch weitere elektronenziehende Reste, wenn die Zahl der freien Valenzen an Ar¹ dies zulässt. Bevorzugt wird A¹ gewählt aus Benzolsystemen, Naphthalinsystemen, Anthracensystemen, Phenanthrensystemen, Pyridinsystemen, Chinolinsystemen, Isochinolinsystemen, Acridinsystemen, Pyrazinsystemen, Pyridazinsystemen, Pyrimidinsystemen, Chinazolinsystemen, Chinooxalinsystemen und Triazinsystemen. Ganz besonders bevorzugt sind Benzolsysteme, Naphthalinsysteme und 1,3,5-Triazinsysteme.
- X: ist Wasserstoff oder ein elektronenziehender Rest, d.h. ein Rest, der einen -I-Effekt oder einen -M-Effekt oder beides zeigt, bevorzugt gewählt aus NO₂-Gruppen, CF₃-Gruppen und ganz besonders bevorzugt aus SO₃M-Gruppen
- Y: ist eine bivalente funktionelle Gruppe oder eine Einfachbindung oder eine C₂-C₂₀-Alkylengruppe, bei der bis zu 3 nicht-benachbarte CH₂-Gruppen durch Sauerstoff oder -NR³- ersetzt sein können. Beispiele für bivalente funktionelle Gruppen sind -O-, -S-. Bevorzugte bivalente funktionelle Gruppen sind die Gruppen Y.1 bis Y.9 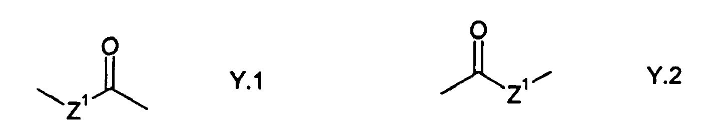 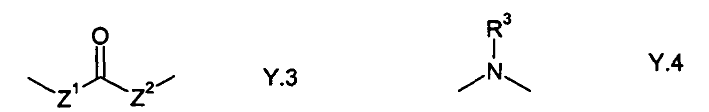 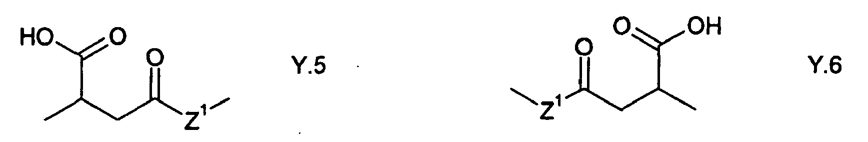 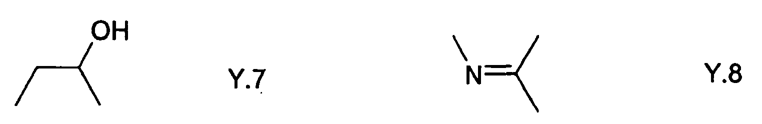 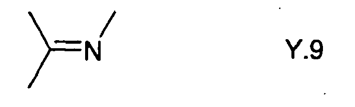
wobei
Z¹ und Z² gleich oder verschieden sein können und gewählt aus Sauerstoff und N-R³.

Eine andere bevorzugte bivalente funktionelle Gruppe Y ist die Diazogruppe -N=N- für den Fall, dass X Wasserstoff bedeutet.

Beispiele für C₂-C₂₀-Alkylengruppen, in denen bis zu 3 nicht-benachbarte CH₂-Gruppen durch NR³ oder Sauerstoff ersetzt sein können, sind -CH₂-CH₂-, -C(CH₃)₂-, -CH₂-CH(CH₃)-, -(CH₂)₃-, -CH₂-CH(C₂H₅)-, -(CH₂)₄-, -(CH₂)₅-, -(CH₂)₆-, -(CH₂)₇-, -(CH₂)₈-, -(CH₂)₉-, -(CH₂)₁₀-; -(CH₂)₂₀-, -CH₂-, -CH₂-CH₂-, -C(CH₃)₂-, -CH₂CH(CH[CH₃]₂)-, -CH₂-CH(n-C₃H₇)-. -[CH(CH₃)]₂-, -CH(CH₃)-CH₂-CH₂-CH(CH₃)-, -CH(CH₃)-CH₂-CH(CH₃)-, -CH₂-C(CH₃)₂-CH₂-, -CH₂-CH(n-C₄H₉)-, -CH₂-CH(t-C₄H₉)-, -CH₂-CH(C₂H₅)-, -CH₂-O-, -CH₂-O-CH₂-, -CH₂-NH-, -CH₂-N(CH₃)-, -(CH₂)₃-NH-(CH₂)₂-NH-, -(CH₂)₂-O-(CH₂)₂-, -[(CH₂)-O]₂-(CH₂)₂-, -[(CH₂)₂-O]₃-(CH₂)₂-.
- R³: sind gleich oder verschieden und unabhängig voneinander gewählt aus Wasserstoff, Phenyl oder
C₁-C₁₀-Alkyl, verzweigt oder unverzweigt, wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl und ganz besonders bevorzugt Methyl.
- B: gewählt aus -C(R¹)=CH₂ und CHR¹-CH₂-Q, wobei
- R¹: gewählt wird aus Phenyl, C₁-C₁₀-Alkyl, verzweigt oder unverzweigt, wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl und insbesondere Methyl,
und ganz besonders bevorzugt Wasserstoff.
- Q: steht für unter wässrig-alkalischen Bedingungen abspaltbaren Gruppen, beispielsweise Alkoxylatgruppen, Halogenid, bevorzugt sind für O-SO₃M, OSO₂-Ar² oder O-CO-R² steht.
- M: steht für Wasserstoff
oder Alkalimetall wie Li, Na, K, Rb oder Cs, bevorzugt Na oder K, oder Ammonium, ausgewählt aus NH₄, C₁-C₁₀-Alkylammonium, Di-C₁-C₁₀-alkyl-ammonium, Tri-C₁-C₁₀-alkylammonium, Tetra-C₁-C₁₀-alkylammonium, wobei C₁-C₁₀-Alkyl gleich oder verschieden sein können und wie oben stehend definiert, Arylammonium wie beispielsweise Anilinium, Aryldialkylammonium wie beispielsweise Dimethylphenylammonium, ω-Hydroxyalkylammonium wie beispielsweise 2-Hydroxyethylammonium, 3-Hydroxypropylammonium, Di-2-hydroxyalkylammonium, Alkyl-ω-Hydroxyalkylammonium wie beispielsweise N-Methyl-2-Hydroxyethylammonium, N,N-Dimethyl-2-hydroxyethylammonium, N-n-Butyl-2-Hydroxyethylammonium.
- Ar²: steht für Phenyl, unsubstituiert oder ein- oder mehrfach substituiert mit Halogen, NO₂, C₁-C₁₀-Alkyl, das wie oben definiert ist, oder SO₃M Bevorzugt steht Ar² für Phenyl, para-Toluyl und para-Nitrobenryl.
- R²: steht für Wasserstoff, Phenyl oder
C₁-C₁₀-Alkyl, verzweigt oder unverzweigt, wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl; besonders bevorzugt C₁-C₄-Alkyl wie Methyl, Ethyl, n-Propyl, iso-Propyl, n-Butyl, iso-Butyl, sec.-Butyl und tert.-Butyl und ganz besonders bevorzugt Methyl.
- W: ist ein Rest, der von einer Verbindung abgeleitet ist, die auf Leder hydrophobierend, fettend oder gerbend wirkt. Darunter sind solche Gruppen zu verstehen, die - mit Wasserstoff abgesättigt - zu Verbindungen führen, die auf Leder hydrophobierend, fettend oder gerbend wirken. Beispiele für bevorzugte hydrophobierend und/oder fettend wirkende Gruppen sind mono-, di- und polyfunktionale Polysiloxanreste, C₈-C₆₀-Alkylreste, verzweigt oder unverzweigt, beispielsweise Polyisobutylenreste mit 2 bis 15 Isobutyleneinheiten, C₈-C₆₀-Alkyl-eneinheiten, verzweigt oder unverzweigt, mehrfach fluorierte C₁-C₂₀-Alkylreste, beispielsweise CF₃, n-C₄F₉,
CH₂-CH₂-CF₃, -CH₂-CH₂-C₂F₅, CH₂-CH₂-n-C₃F₇, CH₂-CH₂-iso-C₃F₇, CH₂-CH₂-n-C₄F₉, CH₂-CH₂-n-C₆F₁₃, CH₂-CH₂-n-C₈F₁₇, CH₂-CH₂-n-C₁₀F_{21,} CH₂-CH₂-n-C₁₂F₂₅.
Copolymerisate von Maleinsäureanhydrid und C₁₀-C₃₀-α-Olefinen, Copolymerisate von Maleinsäureanhydrid und Polyisobuten mit 2 bis 125, bevorzugt 5 bis 50 Isobutyleneinheiten.

Beispiele für gerbend wirkende Gruppen sind
Polyacrylsäuren,
Polymethacrylsäuren,
Phenolsulfonsäure-Harnstoff-Formaldehyd-Kondensate, vorzugsweise mit einem Molekulargewicht Mₙ im Bereich von 300 bis 100.000 g/mol,
Phenol-Dihydroxydiphenylsulfon-Hamstoff-Kondensate,
Phenol-Dihydroxydiphenylsulfon-Hamstoff-Formaldehyd-Kondensate,
Phenolsulfonsäure-Formaldehyd-Kondensate,
Naphthalinmono- und Naphthalindisulfonsäure-Formaldehyd-Kondensate,
Naphthalinmono- und Naphthalindisulfonsäure-Hamstoff-Formaldehyd-Kondensate,
Melamin-Harnstoff-Formaldehyd-Kondensate,
Melamin-Formaldehyd-Kondensate,
Bisphenol-A-Formaldehyd-Kondensate,
Bisphenol-A-Harnstoff-Formaldehyd-Kondensate.

Ein weiterer Gegenstand der vorliegende Erfindung ist ein Verfahren zur Herstellung von erfindungsgemäßen Verbindungen.

Erfindungsgemäße Verbindungen kann man beispielsweise so herstellen, dass man mindestens eine Verbindung, die mindestens eine Gruppe der allgemeinen Formel II 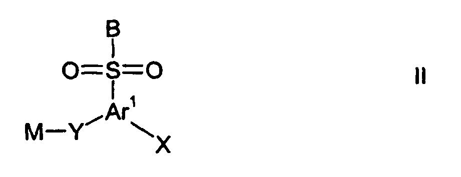 aufweist und in der die Variablen wie oben stehend definiert sind, mit mindestens einer Verbindung W-G umsetzt, die eine funktionelle Gruppe G aufweist, die mit Y-M zur Reaktion befähigt ist. Beispielsweise kann Y gewählt sein aus -O-, -S- oder -NR³ und G aus Wasserstoff, OH, Halogen, insbesondere CI oder Br.

In einer speziellen Variante der vorliegenden Erfindung kann man während der Synthese von erfindungsgemäßen Verbindungen die funktionelle Gruppe Y aufbauen. So ist es beispielsweise möglich, mindestens eine Verbindung einzusetzen, die mindestens eine Gruppe der allgemeinen Formel III 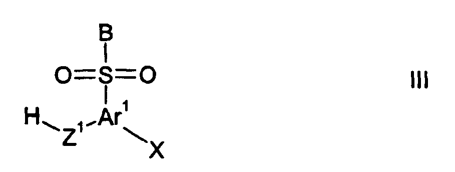 aufweist, mit mindestens einer Verbindung W-CO-G oder W-CO-OCH₃ oder W-Z²-CO-G oder einer Verbindung der Formel IV

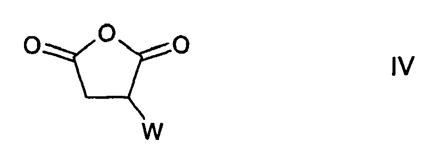

Auch ist es möglich, mindestens eine Verbindung der allgemeinen Formel V a bis V c 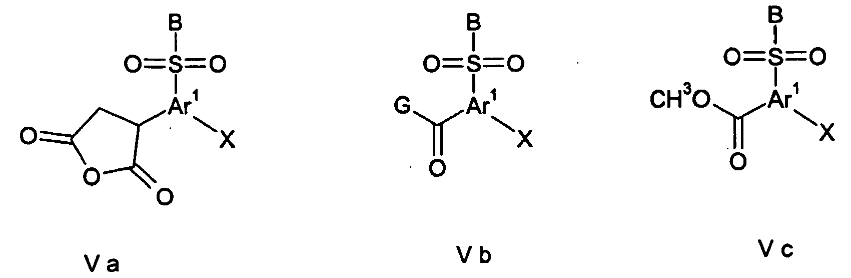 mit einer Verbindung W-Z'-H umzusetzen.

In einer anderen Variante zur Herstellung von erfindungsgemäßer Verbindung setzt man mindestens eine Verbindung der allgemeinen Formel VI 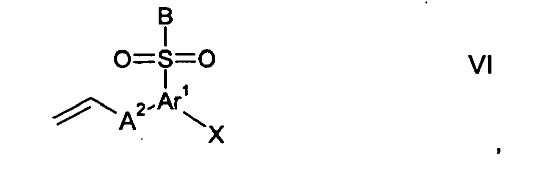 vorzugsweise in Gegenwart eines Hydrosilylierungskatalysators, mit mindestens einem reaktiven Polysiloxan um, das sich durch Substitution von A¹-* durch Wasserstoff in den Polysiloxanresten der Formeln W1.1 bis W1.3, W2.1 bis W2.3 und W3.1 bis W3.3 ableiten lässt.

Dabei steht A² für
eine Einfachbindung oder
C₁-C₁₈-Alkylen, in dem bis zu 3 nicht-benachbarte CH₂-Gruppen durch Sauerstoff oder NR³ ersetzt sein können. Beispielhaft seien genannt: -CH₂-, -CH₂-CH₂-, -C(CH₃)₂-, -CH₂-CH(CH₃)-, -CH₂-CH(CH[CH₃]₂)-, -CH₂-CH(n-C₃H₇)-, -[CH(CH₃)]₂-, -CH(CH₃)-CH₂-CH₂-CH(CH₃)-, -CH(CH₃)-CH₂-CH(CH₃)-, -CH₂-C(CH₃)₂-CH₂-, -CH₂-CH(n-C₄H₉)-, -CH₂-CH(t-C₄H₉)-(CH₂)3-, -CH₂-CH(C₂H₅)-, -(CH₂)₄₋, -(CH₂)₅-, -(CH₂)₆-, -(CH₂)₇-. -(CH₂)₈-, -(CH₂)₉-, -(CH₂)₁₀-; -(CH₂)₁₈-, -CH₂-O-, -CH₂-O-CH₂-, -CH₂-NH-, -CH₂-N(CH₃)-, -(CH₂)₃-NH-(CH₂)₂-NH-, -(CH₂)₂-O-(CH₂)₂-, -[(CH₂)₂-O]₂-(CH₂)₂-, -[(CH₂)₂-O]₃-(CH₂)₂-.

Im folgenden werden Verbindungen der Formel W-CO-G, W-CO-OCH₃, W-Z²-CO-G, W-Z¹-H, Verbindungen der Formel IV und vorstehend genannte reaktive Polysiloxane auch als Derivate von W bezeichnet.

Zur Umsetzung von mindestens einer Verbindung der allgemeinen Formel II, III, V a bis V c oder VI mit mindestens einem Derivat von VI kann man einen oder mehrere Katalysatoren einsetzen, beispielsweise Säuren, schwache Basen oder zum Zwecke einer Hydrosilylierung einen geeigneten Übergangsmetallkatalysator.

Zur Umsetzung von Silikonverbindung mit mindestens einer Verbindung der allgemeinen Formel II, III, V a bis V c oder VI kann man mindestens ein Derivat von W und mindestens eine Verbindung der allgemeinen Formel II, III, V a bis V c oder VI vorzugsweise so dosieren, dass die jeweiligen funktionellen Gruppen jeweils im molaren Verhältnis von 1 : 1 in der jeweiligen Reaktionsmischung vorliegen. Man kann jedoch auch einen der beiden Reaktionspartner - Verbindung der allgemeinen Formel I I, III, V a bis V c oder VI und Derivat von W - im molaren Überschuss dosieren, beispielsweise in 0,1 bis 2 Äquivalenten Überschuss, bezogen auf die jeweiligen funktionellen Gruppen.

In einer Ausführungsform der vorliegenden Erfindung führt man die Herstellung von erfindungsgemäßer Verbindung bei Temperaturen im Bereich von - 20°C bis 200°C durch, bevorzugt bei -10°C bis 140°C.

In einer Ausführungsform der vorliegenden Erfindung führt man die Herstellung von erfindungsgemäßer Verbindung in Gegenwart eines oder mehrerer organischer Lösungsmittel durch, beispielsweise Tetrahydrofuran (THF), Aceton, Methylethylketon oder 1,4-Dioxan. In einer anderen Ausführungsform der vorliegenden Erfindung führt man die Herstellung von erfindungsgemäßer Verbindung ohne Verwendung von Lösungsmittel durch.

In einer Ausführungsform der vorliegenden Erfindung führt man die Herstellung von erfindungsgemäßer Verbindung bei Atmosphärendruck durch. Man kann jedoch auch einen Druck im Bereich von 0,1 bis 0,99 Atmosphären wählen. Man kann auch einen Druck im Bereich von 1,01 bis 20 Atmosphären wählen.

Natürlich ist es im Rahmen der vorliegenden Erfindung auch möglich, mindestens zwei verschiedene Verbindungen der allgemeinen Formel II, III, V a bis V c oder VI mit einem Derivat von W in einer Eintopfreaktion oder nacheinander miteinander umzusetzen. Natürlich ist es im Rahmen der vorliegenden Erfindung ebenfalls möglich, mindestens zwei verschiedene Derivate von W mit einer Verbindung der allgemeinen Formel II, III, V a bis V c oder VI umzusetzen.

Man kann erfindungsgemäße Verbindungen nach der Synthese aufreinigen. In einer anderen Ausführungsform der vorliegenden Erfindung verzichtet man auf das Aufreinigen von erfindungsgemäßen Verbindungen und setzt sie gegebenenfalls im Gemisch mit einem oder mehreren Reaktionspartnern ein.

Wenn man erfindungsgemäßes Verfahren in Gegenwart von einem oder mehreren organischen Lösungsmitteln durchgeführt hat, so kann man nach beendeter Synthese das oder die eingesetzten organischen Lösungsmittel abtrennen, beispielsweise durch Abdestillieren.

Zur Durchführung des erfindungsgemäßen Verfahrens kann man eine oder mehrere erfindungsgemäße Verbindungen einsetzen, und zwar in Substanz oder vorzugsweise als wässrige Dispersion. Wässrige Dispersionen, enthaltend eine oder mehrere erfindungsgemäße Verbindungen, sind daher ebenfalls Gegenstand der vorliegenden Erfindung.
Erfindungsgemäße wässrige Dispersionen können neben mindestens einer erfindungsgemäßen Verbindung weitere Bestandteile aufweisen, beispielsweise einen oder mehrere Emulgatoren, die anionisch, kationisch, nicht-ionisch oder zwitterionisch sein können.

Geeignete nichtionische Emulgatoren sind z.B. ethoxylierte Mono-, Di- und Tri-Alkylphenole (Ethoxylierungsgrad: 3 bis 50, Alkylrest: C₄-C₁₂) sowie ethoxylierte Fettalkohole (Ethoxylierungsgrad: 3 bis 80; Alkylrest: C₈-C₃₆). Beispiele sind die Lutensol^{®}-Marken der BASF Aktiengesellschaft und die Triton^{®}-Marken der Union Carbide.

Geeignete anionische Emulgatoren sind z.B. Alkalimetall- und Ammoniumsalze von Alkylsulfaten (Alkylrest: C₈ bis C₁₂), von Schwefelsäurehalbestern ethoxylierter Alkanole (Ethoxylierungsgrad: 4 bis 30, Alkylrest: C₁₂-C₁₈) und ethoxylierter Alkyl-phenole (Ethoxylierungsgrad: 3 bis 50, Alkylrest: C₄-C₁₂), von Alkylsulfonsäuren (Alkylrest: C₁₂-C₁₈), von Alkylarylsulfonsäuren (Alkylrest: C₉-C₁₈), und von Sulfosuccinaten wie beispielsweise Sufobemsteinsäuremono- und -diestem.

Geeignete kationische Emulgatoren sind in der Regel einen C₆-C₁₈-Alkyl-, -Aralkyl- oder heterocyclischen Rest aufweisende primäre, sekundäre, tertiäre oder quartäre Ammoniumsalze, Alkanolammoniumsalze, Pyridiniumsalze, Imidazoliniumsalze; Oxazoliniumsalze, Morpholiniumsalze, Thiazoliniumsalze sowie Salze von Aminoxiden, Chinoliniumsalze, Isochinoliniumsalze, Tropyliumsalze, Sulfoniumsalze und Phosphoniumsalze. Beispielhaft genannt seien Dodecylammoniumacetat oder das entsprechende Hydrochlorid, die Chloride oder Acetate der verschiedenen 2-(*N,N,N*-Trimethylammonium)ethylparaffinsäureester, *N*-Cetylpyridiniumchlorid, *N*-Laurylpyridiniumsulfat sowie *N*-Cetyl-*N*,*N*,*N*-trimethylammoniumbromid, *N*-Dodecyl-*N*,*N*,*N*-trimethylammoniumbromid, *N*,*N*-Distearyl-*N*,*N*-dimethylammoniumchlorid sowie das Gemini-Tensid *N*,*N*'-(Lauryldimethyl)ethylendiamindibromid. Zahlreiche weitere Beispiele finden sich in H. Stache, Tensid-Taschenbuch, Carl-Hanser-Verlag, München, Wien, 1981 und in McCutcheon's, Emulsifiers & Detergents, MC Publishing Company, Glen Rock, 1989.

Besonders gut geeignete Emulgatoren sind N-acylierte Aminosäurederivate beispiele weise der Formel VII 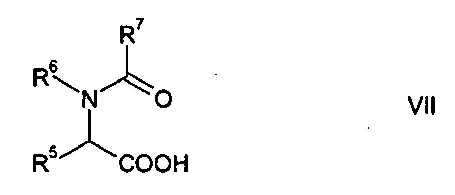 in denen die Variablen wie folgt definiert sind:
R⁵ Wasserstoff,
C₁-C₄-Alkyl, wie Methyl, Ethyl, n-Propyl, iso-Propyl, n-Butyl, iso-Butyl, sec.-Butyl und tert.-Butyl, insbesondere Methyl;
C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
R⁶ C₁-C₄-Alkyl, wie Methyl, Ethyl, n-Propyl, iso-Propyl, n-Butyl, iso-Butyl, sec.-Butyl und tert.-Butyl; insbesondere Methyl;

Die Gruppe CO-R⁷ ist vorzugsweise von gesättigten oder ungesättigten Fettsäuren abgeleitet. Unter gesättigten Fettsäuren sind Carbonsäuren mit C₉-C₂₀-Alkyl-gruppen zu verstehen, die linear oder verzweigt sein können, substituiert oder unsubstituiert. R⁶ kann beispielsweise sein: n-Nonyl, n-Decyl, n-Dodecyl, n-Tetradecyl, n-Pentadecyl, n-Octadecyl, n-Eicosyl.

CO-R⁷ kann von einer ungesättigten Fettsäure mit 9 bis 20 C-Atomen und einer bis 5 C-C-Doppelbindungen abgeleitet sein, wobei die C-C-Doppelbindungen beispielsweise isoliert oder allylisch sein können, beispielsweise der Acylrest der Linolsäure, der Linolensäure, und ganz besonders bevorzugt der Ölsäure.

In einer Ausführungsform der vorliegenden Erfindung sind alle oder zumindest ein gewisser Anteil, beispielsweise ein Drittel oder die Hälfte, der Carboxylgruppen in als Emulgatoren eingesetzten N-acylierten Aminosäurederivaten der Formel IV neutralisiert. Zur Neutralisation eignen sich beispielsweise basische Salze wie Hydroxide oder Carbonate der Alkalimetalle wie beispielsweise Na oder K. Zur Neutralisation eignen sich weiterhin Ammoniak, Alkylamine wie beispielsweise Methylamin, Dimethylamin, Trimethylamin, Ethylamin, Diethylamin, Triethylamin, Ethylendiamin, und ganz besonders Alkanolamine wie beispielsweise Ethanolamin, Diethanolamin, Triethanolamin, N-Methyl-Ethanolamin, N-Methyldiethanolamin oder N-(n-Butyl)-diethanolamin.

Als beispielhafte Vertreter für Verbindungen der Formel VII seien N-Oleylsarcosin, N-Stearylsarcosin, N-Lauroylsarcosin und N-Isononanoylarcosin sowie die jeweiligen Ethanolammoniumsalze, Diethanolammoniumsalze sowie N-Methyldiethanolammoniumsalze genannt.

Andere besonders geeignete Beispiele für Emulgatoren sind solche der allgemeinen Formel VIII 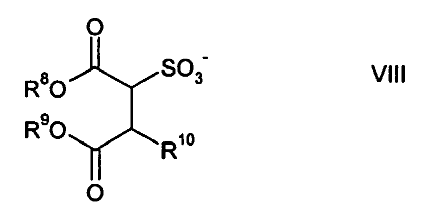 in denen die Variablen wie folgt definiert sind:
- R⁸, R⁹: gleich oder vorzugsweise verschieden und gewählt aus Wasserstoff,
C₁-C₃₀-Alkyl, verzweigt oder unverzweigt, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, n-Undecyl, n-Dodecyl, n-Tridecyl, n-Tetradecyl, n-Hexadecyl, n-Octadecyl, n-Eicosyl, bevorzugt in β-Stellung verzweigte Reste der Formel VIII a 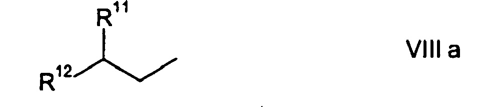 -(CH₂CH₂O)ₓ-O-R¹³ oder -[CH(CH₃)CH₂O)ₓ-O-R¹³, wobei x eine ganze Zahl im Bereich von 1 bis 20 ist,
C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;
- R¹⁰: ist gewählt aus C₁-C₄-Alkyl wie Methyl, Ethyl, n-Propyl, iso-Propyl, n-Butyl, isoButyl, sec.-Butyl, tert.-Butyl
und insbesondere Wasserstoff;
- R¹¹, R¹²: gleich oder vorzugsweise verschieden und gewählt aus C₁-C₂₇-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, n-Undecyl, n-Dodecyl, n-Tridecyl, n-Tetradecyl, n-Hexadecyl, n-Octadecyl, n-Eicosyl;
wobei die Summe der C-Atome von R¹¹ und R¹² maximal 30 beträgt. Vorzugsweise hat
- R¹¹: zwei C-Atome mehr als R¹² ; bevorzugt sind beispielsweise die Kombinationen
R¹¹ = n-Undecyl und R¹² = n-Nonyl,
R¹¹ = n-Dodecyl und R¹² = n-Decyl,
R¹¹ = n-Tridecyl und R¹² = n-Undecyl,
R¹¹ = n-Tetradecyl und R¹² = n-Dodecyl,
R¹¹ = n-Pentadecyl und R¹² = n-Tridecyl.
- R¹³: ist gewählt aus C₁-C₄-Alkyl wie Methyl, Ethyl, n-Propyl, iso-Propyl, n-Butyl,
iso-Butyl, sec.-Butyl, tert.-Butyl,
Phenyl, ortho-Tolyl, meta-Tolyl, para-Tolyl
und insbesondere Wasserstoff.

In einer bevorzugten Ausführungsform der vorliegenden Erfindung ist genau einer der Reste R⁸ und R⁹ Wasserstoff und der andere Rest gewählt aus C₁-C₃₀-Alkyl.

In einer besonders bevorzugten Ausführungsform der vorliegenden Erfindung wählt man ein Gemisch von mehreren Emulgatoren beispielsweise der Formel VIII, die sich beispielsweise dadurch unterscheiden können, dass in der ersten Verbindung der Formel VIII R⁸ gleich Wasserstoff und R⁹ aus C₁-C₃₀-Alkyl gewählt wird und in der zweiten R⁹ gleich Wasserstoff und R⁸ aus C₁-C₃₀-Alkyl gewählt wird.

In einer Ausführungsform der vorliegenden Erfindung sind alle oder zumindest ein gewisser Anteil, beispielsweise ein Drittel oder die Hälfte, der Sulfonylgruppen in Emulgatoren der Formel VIII neutralisiert. Zur Neutralisation eignen sich beispielsweise basische Salze wie Hydroxide oder Carbonate der Alkalimetalle wie beispielsweise Na oder K. Zur Neutralisation eignen sich weiterhin Ammoniak, Alkylamine wie beispielsweise Methylamin, Dimethylamin, Trimethylamin, Ethylamin, Diethylamin, Triethylamin, Ethylendiamin, und ganz besonders Alkanolamine wie beispielsweise Ethanolamin, Diethanolamin, Triethanolamin, N-Methyl-Ethanolamin, N-Methyldiethanolamin oder N-(n-Butyl)-diethanolamin.

Die Herstellung von Verbindungen der Formel VIII ist an sich bekannt und in WO 01/68584 beschrieben. Sie gelingt beispielsweise durch einfache oder doppelte Veresterung von Dicarbonsäureanhydriden der allgemeinen Formel IX 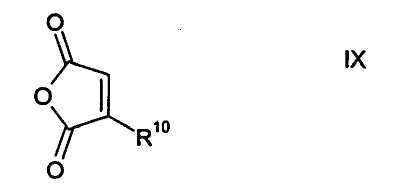 mit entsprechenden Alkoholen, die nicht rein vorliegen müssen, gefolgt von einer Umsetzung mit Disulfit, beispielsweise Na₂S₂O₅.

Erfindungsgemäße Dispersionen können anstatt aufgereinigter Emulgatoren, beispielsweise der Formel VIII, Gemische von verschiedenen Emulgatoren enthalten. Beispielsweise ist es möglich, zur Veresterung das als Oxoöl 135 oder Oxodicköl 135 (WO 01/68584) bekannte Gemisch zuzusetzen und die so erhältlich Mischung von Estern als Emulgator zu verwenden.

In einer Ausführungsform der vorliegenden Erfindung können in erfindungsgemäßen Dispersionen enthaltene Emulgatoren bis zu 40 Gew.-%, bevorzugt bis zu 20 Gew.-%, bezogen auf Emulgator, mindestens eines Alkohols der Formel X 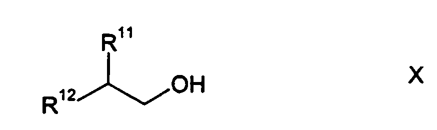 enthalten, wobei in Formel X die Variablen R¹¹ und R¹² wie oben stehend definiert sind.

In einer bevorzugten Ausführungsform der vorliegenden Erfindung können in erfindungsgemäßen Dispersionen enthaltene Emulgatoren bis zu 40 Gew.-%, besonders bevorzugt bis zu 20 Gew.-% Mischungen enthalten, die mindestens einen Alkohol der allgemeinen Formel X umfassen; beispielhaft für derartige Mischungen sei Oxoöl 135 und Oxoöl 13 genannt.

Erfindungsgemäße wässrige Dispersionen enthalten weiterhin Wasser, das entionisiert oder auch salzhaltig sein kann.

In einer Ausführungsform der vorliegenden Erfindung enthalten erfindungsgemäße wässrige Dispersionen mindestens eine weitere hydrophobe Verbindung. Bei mindestens einer weiteren hydrophoben Verbindung handelt es sich dabei um eine Verbindung auf Kohlenwasserstoffbasis, beispielsweise natürliches oder synthetisches Wachs, natives oder synthetisches Öl oder natives oder synthetisches Fett.

Als Beispiele für natürliche Wachse seien Bienenwachs, Korkwachs, Montanwachse oder Camaübawachs genannt.

Als Beispiele für synthetische Wachse seien Polyethylenwachse oder Ethylencopolymerwachse genannt, wie sie beispielsweise durch radikalische Polymerisation von Ethylen oder radikalische Copolymerisation von Ethylen mit beispielsweise (Meth)acrylsäure oder durch Ziegler-Natta-Katalyse erhältlich sind. Weiterhin seien Polyisobutylenwachse genannt. Weiterhin seien Paraffingemische genannt; darunter sind Gemische von Kohlenwasserstoffen zu verstehen, die 12 oder mehr Kohlenstoffatome aufweisen und üblicherweise einen Schmelzpunkt im Bereich von 25 bis 45°C aufweisen. Derartige Paraffingemische können beispielsweise in Raffinerien oder Crackem anfallen und sind als Paraffingatsch und Sasolwachse bekannt. Ein weiteres Beispiel für synthetische Wachse sind Montanesterwachse.

Als Beispiele für natürliche Öle seien bei Zimmertemperatur flüssige Triglyceride genannt, beispielsweise Fischöl, Klauenöl wie beispielsweise Rinderklauenöl, Olivenöl, Baumwollsamenöl, Rapsöl, Nerzöl, Rizinusöl, Sonnenblumenöl und Erdnussöl genannt.

Als Beispiele für synthetische Öle seien Weißöl, Paraffinöl, funktionalisierte Paraffine wie beispielsweise chlorierte oder sulfochlorierte Paraffine oder auch Polyalkylenglykole wie beispielsweise Polyethylenglykol genannt.

Als Beispiele für natürliche Fette seien bei Zimmertemperatur feste native Triglyceride genannt wie beispielsweise Lanolin, Schellackwachs sowie deren Gemische.

In einer bevorzugten Ausführungsform der vorliegenden Erfindung handelt es sich bei der weiteren hydrophoben Verbindung um mindestens ein natives Triglycerid.

In einer weiteren bevorzugten Ausführungsform setzt man eine Kombination aus mindestens einem bei Zimmertemperatur festen oder flüssigen nativen Triglycerid sowie einem Paraffingemisch mit einem Schmelzpunkt im Bereich von 25 bis 40°C ein. Das Mengenverhältnis ist an sich unkritisch, geeignet sind Gewichtsverhältnisse natives Triglycerid : Paraffingemisch im Bereich von 10 : 1 bis 1 : 10.

Erfindungsgemäße wässrige Dispersionen können weiterhin Zuschlagstoffe enthalten, beispielsweise Biozide, Komplexiermittel, Dispergatoren, Oxidationsstabilisatoren.

In einer Ausführungsform der vorliegenden Erfindung enthalten erfindungsgemäße wässrige Dispersionen
0,1 bis 80 Gew.-%, bevorzugt 5 bis 60 Gew.-% mindestens einer erfindungsgemäßen Verbindung,
0 bis 20 Gew.-%, bevorzugt bis 10 Gew.-%, beispielsweise 0,1 bis 10 Gew.-% mindestens eines Emulgators,
0 bis 40 Gew.-%, bevorzugt bis 30 Gew.-%, beispielsweise 0,1 bis 30 Gew.-% mindestens einer weiteren hydrophoben Verbindung,
insgesamt 0 bis 9 Gew.-%, bevorzugt bis 5 Gew.-% an einem oder mehreren weiteren Zuschlagstoffen,
der Rest ist Wasser.

Erfindungsgemäße wässrige Dispersionen zeichnen sich durch gute Lagerstabilität aus.

Zur Herstellung von erfindungsgemäßen wässrigen Dispersionen kann man beispielsweise so vorgehen, dass man mindestens eine erfindungsgemäße Verbindung mit Wasser und gegebenenfalls mit mindestens einem Emulgator, mindestens einer weiteren hydrophoben Verbindung und gegebenenfalls mit mindestens einem Zuschlagstoff vermischt. Zum Vermischen sind übliche Vermischungsapparaturen geeignet, beispielsweise Rührgefäße, Schüttelgefäße und Ultraturrax-Rührer. Wenn es gewünscht ist, kann man im Anschluss an das Vermischen mit Hilfe eines Spalthomogenisators homogenisieren.

In einer speziellen Variante kann man so vorgehen, dass man mindestens eine erfindungsgemäße Verbindung mit Wasser, das eine Temperatur im Bereich von 60 bis 95°C, bevorzugt von 75 bis 85°C und ganz besonders bevorzugt auf etwa 80°C erwärmt ist, und gegebenenfalls mit mindestens einem Emulgator, mindestens einer weiteren hydrophoben Verbindung und gegebenenfalls mit mindestens einem Zuschlagstoff vermischt.

In einer anderen Variante löst man mindestens eine erfindungsgemäße Verbindung in mindestens einem organischen Lösungsmittel, beispielsweise THF, Aceton, Isopropanol oder Methylethylketon, vermischt gegebenenfalls mit mindestens einem Emulgator, mindestens einer weiteren hydrophoben Verbindung und gegebenenfalls mit mindestens einem Zuschlagstoff und trennt das oder die organischen Lösungsmittel ab, beispielsweise durch Abdestillieren.

Ein weiterer Gegenstand der vorliegenden Erfindung ist die Verwendung von erfindungsgemäßen wässrigen Dispersionen zur Herstellung von Leder oder Pelzfellen. Ein weiterer Gegenstand der vorliegenden Erfindung ist ein Verfahren zur Herstellung von Leder oder Pelzfellen unter Verwendung von erfindungsgemäßen wässrigen Dispersionen. Ein weiterer Gegenstand der vorliegenden Erfindung sind Leder bzw. Pelzfelle, hergestellt unter Verwendung von erfindungsgemäßen Verbindungen und insbesondere von erfindungsgemäßen wässrigen Dispersionen.
Die Erfindung wird durch Arbeitsbeispiele erläutert.

Allgemeine Bemerkungen:

### I. Herstellung erfindungsgemäßer Verbindungen

### I.1. Herstellung von erfindungsgemäßer Verbindung 1.1

In einem 0,5-I-4-Halskolben wurden 11,3 g (40 mmol) 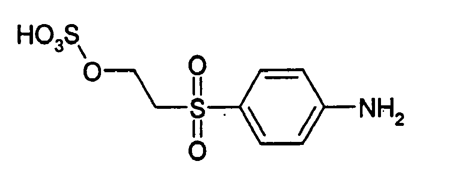 (Parabase) mit 100 g Dimethylsulfoxid (DMSO) versetzt und unter Rühren auf 50°C erwärmt. Anschließend gab man 71,6 g eines Silikonöls, das pro Molekül zwei Gruppen der Formel 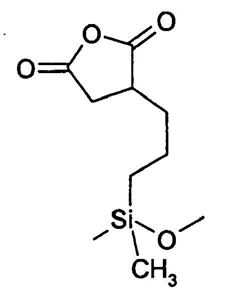 aufweist und bei dem alle Gruppen R⁴ für Methyl stehen, mit einer kinematischen Viskosität von 190 mm²/s, bestimmt nach ISO 3219-3, tropfenweise zu. Nach beendeter Zugabe rührte man 8 Stunden bei 80°C, bis sich durch Titration keine Anhydridgruppen mehr detektieren ließen. Anschließend destillierte man das DMSO bei 80°C und einem Druck von 5 mbar ab. Man erhielt 76,3 g (92 %) eines zähen Öls. Die Struktur der neu synthetisierten Struktureinheiten wurde mit Hilfe von ¹H-NMR-Spektroskopie zu 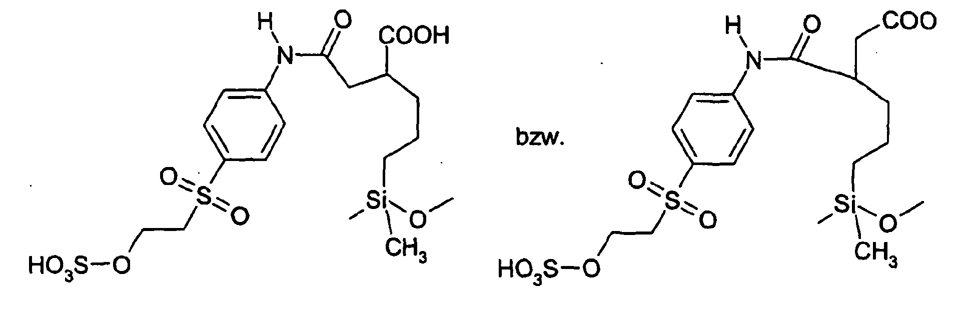 bestimmt.

### 1.2. Herstellung von erfindungsgemäßer Verbindung 1.2

In einem 2-I-4-Halskolben wurden 60,6 g (40 mmol) Stearinsäurechlorid in 300 g Wasser aufgeschlämmt und auf 0°C abgekühlt. Es wurden 62,4 g (0,22 mol) 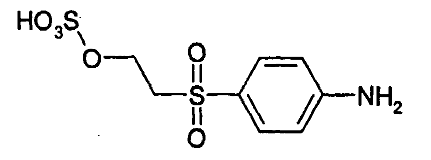 (Parabase) in 500 g Wasser suspendiert und durch Zugabe von Natriumhydrogencar bonat ein pH-Wert von 3,5 eingestellt. Es wurde auf 35°C erwärmt, wobei die Parabase in Lösung ging. Die Parabaselösung wurde in die Stearinsäurechlorid-Aufschlämmung unter Eiskühlung innerhalb von 45 Minuten zugetropft, wobei die Temperatur nicht über 5°C stieg. Dabei wurde 15 Gew.-% Na₂CO₃-Lösung zugegeben, so dass der pH-Wert im Bereich von 4 bis 4,5 blieb. Nach 2 Std. bei 5°C wurde der durch Zugabe von 15 Gew.-% Na₂CO₃-Lösung der pH-Wert auf 5,5 und die Temperatur langsam auf 50°C erhöht. Danach wurde 15 Stunden bei 45°C nachgerührt. Der Umsatz wurde mittels Chlorid-titer und Dünnschichtchromatographie (DC-Analytik: Laufmittel 1 Gew.-Teil Wasser, 155 Gew.-Teile Ethylacetat, Gew.-200 Teile 1-Propanol, 5 Gew.-Teile Essigsäure) bestimmt und entsprach 99%. 70 g Kaliumchlorid wurden zugegeben und das ausgefällte Produkt über eine Fritte filtriert und bei 40°C mit 3 mbar im Vakuumtrockenschrank getrocknet. Es wurden 143 g erfindungsgemäße Verbindung 1.2 erhalten.

Die Struktur der erfindungsgemäße Verbindung 1.2 wurde mit Hilfe von ¹H-NMR-Spektroskopie und IR-Spektroskopie zu

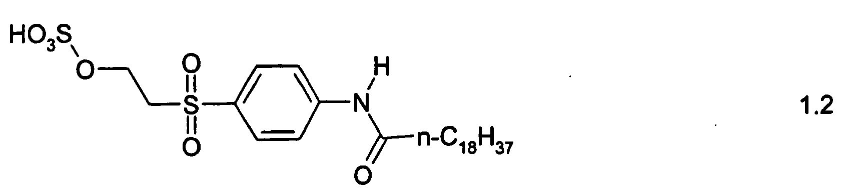

### I.3 Herstellung von erfindungsgemäßer Verbindung 1.3

In einem 2-I-4-Halskolben wurden 60,1 g (40 mmol) Ölsäurechlorid in 300 g Wasser suspendiert und auf 0°C abgekühlt. Es wurden 62,4 g (0,22 mol) 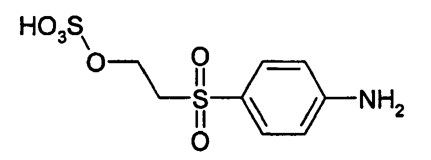 (Parabase) in 500 g Wasser suspendiert und durch Zugabe von Natriumhydrogencar bonat ein pH-Wert von 3,5 eingestellt. Es wurde auf 35°C erwärmt, wobei die Parabase in Lösung ging. Die Parabaselösung wurde in die Stearinsäurechlorid - Suspension unter Eiskühlung innerhalb von 45 Minuten zugetropft, wobei die Temperatur nicht über 5°C stieg. Dabei wurde 15 Gew.-% Na₂CO₃-Lösung zugegeben, so dass der pH-Wert im Bereich von 4 bis 4,5 blieb. Nach 2 Std. bei 5°C wurde der durch Zugabe von 15 Gew.-% Na₂CO₃-Lösung der pH-Wert auf 5,5 und die Temperatur langsam auf 50°C erhöht. Danach wurde 15 Stunden wurde bei 45°C nachgerührt. Der Umsatz wurde mittels Chlorid-titer und Dünnschichtchromatographie (DC-Analytik: Laufmittel 1 Gew.-Teil Wasser, 155 Gew.-Teile Ethylacetat, Gew.-200 Teile 1-Propanol, 5 Gew.-Teile Essigsäure) bestimmt und entsprach 99%. 70 g Kaliumchlorid wurden zugegeben und das ausgefällte Produkt über eine Fritte filtriert und bei 40°C mit 3 mbar im Vakuumtrockenschrank getrocknet. Es wurden 143 g erfindungsgemäße Verbindung 1.3 erhalten.

Die Struktur der erfindungsgemäße Verbindung 1.3 wurde mit Hilfe von ¹H-NMR-Spektroskopie und IR-Spektroskopie zu 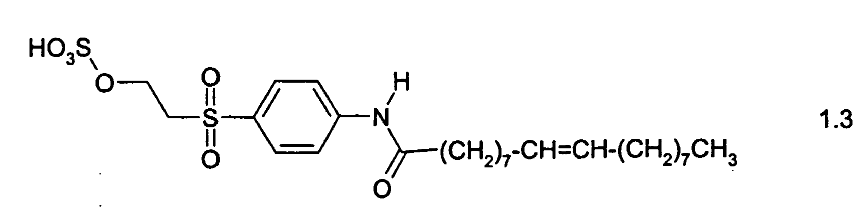 bestimmt, wobei die C-C-Doppelbindung (E)-ständig substituiert war.

### 1.4 Herstellung von erfindungsgemäßer Verbindung 1.4.

### I.4.1 Herstellung von Komponente A

In einem 1-I-Kolben wurden 102 g (1,08 mol) Phenol vorgelegt und langsam 124,2 g (1,21 mol) konzentrierte Schwefelsäure zugetropft. Danach wurde 2 Stunden bei 100 bis 105°C gerührt. Anschließend wurde über einen Zeitraum von 30 Minuten bei 55°C 141,5 g (1,18 mol) wässrige Harnstofflösung (50 Gew.-%) zugegeben. Die Temperatur wurde auf 85°C erhöht und nach 5 Minuten auf 70°C eingestellt. Dann tropfte man innerhalb 2 Stunden 206 g (2,06 mol) wässrige Formaldehydlösung (30 Gew.-%) zu. Nach weiteren 5 Minuten Rühren bei 70°C wurden 19,1 g (0,24 mol) Natronlauge (50 Gew.-%) und 15 ml Wasser zugegeben. Bei 53°C wurden 67,9 g (0,72 mol) Phenol und 1,20 g Ethylendiamintetraessigsäure-Natriumsalz zugefügt, 10 Minuten bei 51 bis 55°C gerührt und schließlich unter Erwärmung auf 60°C über einen Zeitraum von 30 Minuten 56,8 g (0,57 mol) wässrige Formaldehydlösung (30 Gew.-%) zugegeben. Nach 5 Minuten Nachrührzeit bei 60°C wurde mit ca. 95 g (1,18 mol) Natronlauge (50Gew.-%) auf einen pH-Wert von ca. 7 eingestellt und auf Raumtemperatur abgekühlt. Man erhielt etwa 900 g Lösung.

### I.4.2 Herstellung von Komponente B

In einem Rundkolben wurden 62,4 g (0,20 mol) Parabase vorgelegt und in 500 ml demineralisiertem Wasser aufgeschlämmt. Bei 35 bis 40°C wurden 17,5 g (0,21 mol) Natriumhydrogencarbonat in Portionen zugegeben, so dass sich ein pH-Wert von 3,5 bis 4,0 einstellte. Durch Zugabe von 147 g (0,30 mol) Schwefelsäure (20%ig) wurde die Parabase sauer ausgefällt. Anschließend wurden 140 g Eis zugegeben und die Temperatur der Lösung auf 5°C gebracht. Bei dieser Temperatur wurden über einen Zeitraum von 15 Minuten 62,0 ml (0,21 mol) wässriger Natriumnitritlösung (3,33 molar) zudosiert und anschließend 1 Stunde bei 5-10°C gerührt. Der Überschuss an Natriumnitrit wurde mit 1,3 g Amidosulfonsäure vernichtet und das Produkt unter 5°C gelagert. Man erhielt ca. 920 g Lösung.

### I.4.3 Kupplung von Komponente A und Komponente B

In einem Rundkolben wurden 920 g von Komponente B vorgelegt, bei 5°C langsam mit 251 g Komponente A versetzt. Dabei lag der pH-Wert unter 2,0. Anschließend wurde über einen Zeitraum von 2 Stunden 150 g (0,21 mol) Natriumcarbonatlösung (15%ig) zudosiert, dabei stieg der pH-Wert auf ca. 6 an, die Temperatur blieb unter 10°C. Nach 3 Stunden Nachrühren bei 10°C wurde auf Raumtemperatur erwärmt. Man erhielt ca. 1320 g Lösung mit einem Feststoffgehalt von 16%.

### II. Herstellung von erfindungsgemäßen wässrigen Dispersionen

### II.1 Herstellung der erfindungsgemäßen wässrigen Dispersion D1

Man verrührte in einem 1-I-Becherglas 45 g Verbindung 1.1 und 2,3 g n-C₁₈H₃₇O(CH₂CH₂₀O)₂₅-H mit 52,7 g destilliertem Wasser bei 80°C (20000 Umdrehungen/min) und kühlte anschließend auf 20°C ab. Man erhielt erfindungsgemäße Dispersion D1.

### II.2 Herstellung der erfindungsgemäßen wässrigen Dispersion D2

Man verrührte in einem 1-I-Becherglas 40 g Verbindung 1.2 und 2 g n-C₁₈H₃₇O(CH₂CH₂₀O)₂₅-H mit 58 g destilliertem Wasser bei 80°C (20000 Umdrehungen/min) und kühlte anschließend auf 20°C ab. Man erhielt erfindungsgemäße Dispersion D2.

### II.3 Herstellung der erfindungsgemäßen wässrigen Dispersion D3

Man verrührte in einem 1-I-Becherglas 40 g Verbindung 1.3 und 2 g n-C₁₉H₃₇O(CH₂CH₂₀O)₂₅-H in 58 g destilliertem Wasser bei 80°C (2000 U/min) und kühlte anschließend auf 20°C ab. Man erhielt erfindungsgemäße Dispersion D3.

### III. Herstellung von erfindungsgemäßem Leder

Die Angaben in Gewichtsteilen beziehen sich jeweils auf das Falzgewicht, wenn nicht anders angegeben. Bei allen Operationen wurde das Fass (Wackerfass) etwa 10 mal pro Minute gedreht, wenn nicht anders angegeben.

Die Bestimmung der Echtheiten erfolgte nach den folgenden, international anerkannten Normen:

| | |
|---|---|
| Schweißechtheit: | in Anlehnung an Veslic C4260 |
| Waschechtheit: | in Anlehnung an DIN EN ISO 15703 |
| Migrationsechtheit: | in Anlehnung an DIN EN ISO 15701, sowie durch 16 stündiges Lagern bei 85 °C in einer Feuchte von 95 % unter ansonsten analogen Bedingungen zu DIN EN ISO 15701 |
| Reibechtheit: | in Anlehnung an DIN EN ISO 105 - X12 (Crockmeter, Reibung mit Baumwollgewebe) sowie in Anlehnung an DIN EN ISO 11640 (Veslic, Reibung mit Filz) |

### III.1 Herstellung von erfindungsgemäßem Leder L3.1

a) Ein Lederstück von 100 Gewichtsteilen eines mit Glutardialdehyd gegerbten Rindsleders der Falzstärke 1,1 mm wurde in einem mit 200 Gewichtsteilen Wasser gefüllten Wackerfass 20 Minuten bei 30°C gewaschen. Danach wurde mit 150 Gewichtsteilen Wasser, 2 Gewichtsteilen Naphthalinsulfonsäure-Formaldehyd-Kondensationsprodukt, hergestellt nach US 5,186,846, Beispiel "Dispergiermittel 1", sowie 10 Gewichtsteilen Dispersion D2 über einen Zeitraum von 60 Minuten bei einem pH-Wert von 4,4 und 30°C behandelt. Durch portionsweise Zugabe von 100 Gewichtsteilen 15 Gew.-% Sodalösung wurde bei 40°C der pH-Wert zwischen 10,0 und 10,2 zur Fixierung des Fettungsmittels 120 Minuten lang gehalten. Anschließend folgte sechsmal eine je 10 minütige Waschoperation in 200 Gewichtsteilen Wasser bei 40°C. Durch Zugabe von 200 Gewichtsteilen Wasser und 0,7 Gewichtsteilen Ameisensäure wurde ein pH-Wert von 4,7 eingestellt. Man erhielt gefettetes Leder.
b) Das derart gefettete Leder wurde in einer frisch angesetzten Flotte aus 100 Gewichtsteilen Wasser und 3 Gewichtsteilen eines Farbstoffgemisches, das sich seinerseits aus
   70 Gewichtsteilen Farbstoff aus EP-B 0 970 148, Beispiel 2.18 und 30 Gewichtsteilen Acid Brown 75 (Eisenkomplex), Colour Index 1.7.16.
   zusammensetzte, 60 min bei 35°C gefärbt. Anschließend fügte man zur Flotte 15 Gewichtsteile Sulfongerbstoff aus EP-B 0 459 168, Beispiel K1, und 10 Gewichtsteile Vegetabilgerbstoff Tara und walkte 120 Minuten. Schließlich säuerte man mit 2 Gewichtsteilen konzentrierter Ameisensäure auf pH-Wert 3,6 ab und walkte zweimal 10 Minuten und einmal 30 Minuten. Das so gefärbte, nachgegerbte und gefettete Leder wurde noch mit 15°C kalten Wasser 10 Minuten gespült und anschließend ausgereckt, über Nacht bei Raumtemperatur hängegetrocknet, konditioniert, gestollt und gespannt.

Es wurde erfindungsgemäßes braunes Leder L3.1 erhalten, das über hervorragende Waschechtheit, hervorragende Weichheit und Fülle und sehr gute Fogging-Werte verfügte.

### III.2 Herstellung von erfindungsgemäßem Leder L3.2

Ein Lederstück von 100 Gewichtsteilen eines chromgegerbten Rindsleders der Falzstärke 1,8 mm wurde in einem mit 200 Gewichtsteilen Wasser und 0,1 Gewichtsteilen Ameisensäure gefüllten Wackerfass 10 Minuten bei 35°C gewaschen und anschließend in einer aus 100 Gewichtsteilen Wasser, 2 Gewichtsteilen Natriumformiat, 1 Gewichtsteil Natriumacetat, 1,5 Gewichtsteil Natriumbicarbonat und 1 Gewichtsteil eines Naphthalinsulfonsäure-Formaldehyd-Kondensationsprodukt, hergestellt nach US 5,186,846, Beispiel "Dispergiermittel 1", bestehenden Flotte 120 Minuten bei 35°C neutralisiert. Danach wies die Entsäuerungsflotte einen pH-Wert von 6,3 auf. Anschließend wurde mit 200 Gewichtsteilen Wasser 10 Minuten bei 35°C gewaschen.

In einer frisch angesetzten Flotte aus 150 Gewichtsteilen Wasser und 20 Gewichtsteilen erfindungsgemäße Dispersion D3 wurde 30 Minuten bei pH-Wert 5,6 und 30°C behandelt. Durch portionsweise Zugabe von 13 Gewichtsteilen einer 15 Gew.-% Sodalösung stellte man den pH-Wert der Flotte zwischen 8,5 - 9,5 ein und walkte 180 Minuten bei 40°C. Anschließend folgte 3 mal eine 10 minütige Waschoperation in 200 Gewichtsteilen Wasser bei 40°C. Durch Zugabe von 200 Gewichtsteilen Wasser und 1,5 Gewichtsteilen Ameisensäure wurde ein pH-Wert von 4,8 eingestellt. Das derart gefettete Leder wurde mit in einer frisch angesetzten Flotte mit 100 Gewichtsteilen Wasser und 3 Gewichtsteilen eines Farbstoffgemisches, das sich seinerseits aus
70 Gewichtsteilen Farbstoff aus EP-B 0 970 148, Beispiel 2.18 und
30 Gewichtsteilen Acid Brown 75 (Eisenkomplex), Colour Index 1.7.16. zusammensetzte,
60 min bei 35°C gefärbt. Zur Flotte wurden 15 Gewichtsteile Sulfongerbstoff aus EP-B 0 459 168, Beispiel K1 zugesetzt und 60 Minuten gewalkt. Schließlich säuerte man mit 0,5 Gewichtsteile Ameisensäure auf pH-Wert 4,4 ab und walkte einmal 10 Minuten und einmal 30 Minuten. Anschließend säuerte man mit 1,5 Gewichtsteilen konzentrierte Ameisensäure auf pH-Wert 3,5 ab und walkt 40 Minuten. Das derart gefärbte und gefettete Leder wurde noch mit 15°C kaltem Wasser 10 Minuten gespült und anschließend ausgereckt, über Nacht bei Raumtemperatur hängegetrocknet, konditioniert, gestollt und gespannt.

Es wurde erfindungsgemäßes braunes Leder L3.2 erhalten, das über hervorragende Wasch-, Schweiß-, Reib- und Migrationsechtheit, hervorragende Weichheit und Fülle und sehr gute Fogging Werte verfügt.

### III.3 Herstellung von erfindungsgemäßem Leder L3.3

a) Ein Lederstück von 100 Gewichtsteilen eines mit Glutardialdehyd gegerbten Rindsleders der Falzstärke 1,1 mm wurde in einem mit 200 Gewichtsteilen Wasser gefüllten Wackerfass 20 Minuten bei 30°C gewaschen. Danach wurde mit 150 Gewichtsteilen Wasser, 2 Gewichtsteilen eines Naphthalinsulfonsäure-Formaldehyd-Kondensationsprodukts, hergestellt nach US 5,186,846, Beispiel "Dispergiermittel 1", und 3 Gewichtsteilen eines Farbstoffes 5 Minuten gewalkt.

Der Farbstoff war folgendermaßen hergestellt worden
1 mol MSP (3-(2-Sulfatoethylsulfonyl)-6-aminobenzolsulfonsäure) wurde im Eiswasser suspendiert und bei pH-Wert ≤1 und einer Temperatur von 0 bis 5°C mit überschüssigem Natriumnitrit und Salzsäure diazotiert. 1 mol Gammasäure wurde in 25 Gew.-% Natronlauge gelöst und dann durch Zugabe von 21 Gew.-% Salzsäure der pH-Wert unter 1 gestellt. Die Gammasäure fiel aus. Zu dieser Suspension wurde die diazotierte MSP eingestürzt. Die Reaktionsmischung wurde bei pH-Wert ≤1 eine Stunde nachgerührt bis kein diazotiertes MSP bzw. freie Gammasäure mehr nachweisbar war. Danach wurde der pH-Wert durch Zugabe von 15 Gew.-% Natriumcarbonatlösung auf 3 bis 8 angehoben.
b) 1 mol 4,6-Dinitro-2-aminophenol wurde mit überschüssigem Natriumnitrit und Salzsäure bei pH-Wert ≤1 und einer Temperatur von 0 bis 5°C diazotiert und anschließend zu der in Schritt 1) erhaltenen Reaktionsmischung gegeben. Der pH-Wert wurde durch Zugabe von 10 Gew.-% Natronlauge zwischen 3-8 gehalten. Nach beendeter Umsetzung wurde 0,20-0,30 mol Chrom(III)-sulfat zu der Reaktionsmischung gegeben und 1 Stunde nachgerührt. Der Farbstoff wurde durch Zugabe von NaCl ausgesalzen und abgesaugt.

Anschließend wurden 10 Gewichtsteile erfindungsgemäße Dispersion D2 zu der Flotte zugegeben und 60 Minuten bei einem pH-Wert von 4,4 und 30°C behandelt. Durch portionsweise Zugabe von 100 Gewichtsteilen 15 Gew.-% Sodalösung wurde bei 40°C der pH-Wert zwischen 10,0 und 10,2 zur Fixierung 120 Minuten lang gehalten. Anschließend folgte sechsmal eine je 10 minütige Waschoperation in 200 Gewichtsteilen Wasser bei 40°C. Durch Zugabe von 200 Gewichtsteilen Wasser und 0,7 Gewichtsteilen Ameisensäure wurde ein pH-Wert von 4,7 eingestellt.
c) Das derart gefettete und gefärbte Leder wurde in einer frisch angesetzten Flotte aus 100 Gewichtsteilen Wasser und 15 Gewichtsteilen Sulfongerbstoff aus EP-B 0 459 168, Beispiel K1, 10 Gewichtsteilen Vegetabilgerbstoff Tara 120 Minuten nachgegerbt. Danach säuerte man mit 2 Gewichtsteilen konzentrierter Ameisensäure auf einen pH-Wert von 3,6 ab und walkte zweimal 10 Minuten und einmal 30 Minuten. Das erfindungsgemäß gefärbte, nachgegerbte und gefettete Leder wurde noch mit 15°C kalten Wasser 10 Minuten gespült und anschließend ausgereckt, über Nacht bei Raumtemperatur hängegetrocknet, konditioniert, gestollt und gespannt.

Es wurde erfindungsgemäßes Leder L3.3 erhalten, das über hervorragende Farbtiefe, Wasch-, Schweiß-, Reib- und Migrationsechtheit, hervorragende Weichheit und Fülle und sehr gute Fogging-Werte verfügte.

### III.4 Herstellung von erfindungsgemäßem Leder L3.4

a) Ein Lederstück von 100 Gewichtsteilen eines mit Glutardialdehyd gegerbten Rindsleders der Falzstärke 1,1 mm wurde in einem mit 200 Gewichtsteilen Wasser gefüllten Wackerfass 20 Minuten bei 30°C gewaschen. Danach wurde mit 150 Gewichtsteilen Wasser, 2 Gewichtsteilen eines Naphthalinsulfonsäure-Formaldehyd-Kondensationsprodukts, hergestellt nach US 5,186,846, Beispiel "Dispergiermittel 1", und 10 Gewichtsteilen erfindungsgemäßer Verbindung 1.4 60 Minuten bei pH-Wert 4,4 und 30°C nachgegerbt. Durch portionsweise Zugabe von 100 Gewichtsteilen 15 Gew.-% Sodalösung wurde bei 40°C der pH-Wert zwischen 10,0 und 10,2 zur Fixierung des Nachgerbstoffes 120 Minuten lang unter Walken gehalten. Anschließend folgte sechsmal eine je 10 minütige Waschoperation in 200 Gewichtsteilen Wasser bei 40°C. Durch Zugabe von 200 Gewichtsteilen Wasser und 0,7 Gewichtsteilen Ameisensäure wurde ein pH-Wert von 4,7 eingestellt.
b) Das derart nachgegerbte Leder wurde in einer frisch angesetzten Flotte aus 100 Gewichtsteilen Wasser und 3 Gewichtsteilen eines Farbstoffgemisches, das sich seinerseits aus
   70 Gewichtsteilen Farbstoff aus EP-B 0 970 148, Beispiel 2.18 und
   30 Gewichtsteilen Acid Brown 75 (Eisenkomplex), Colour Index 1.7.16.
   zusammensetzte, 60 min bei 35°C gefärbt. Anschließend wurde in der gleichen Flotte mit 10 Gewichtsteilen eines Fettlickers (Fischöl) durch zweistündiges Walken bei 35 °C gefettet. Schließlich säuerte man mit 2 Gewichtsteilen konzentrier ter Ameisensäure auf pH-Wert 3,6 ab und walkte zweimal 10 Minuten und einmal 30 Minuten. Das so gefärbte, nachgegerbte und gefettete Leder wurde noch mit 15°C kalten Wasser 10 Minuten gespült und anschließend ausgereckt, über Nacht bei Raumtemperatur hängegetrocknet, konditioniert, gestollt und gespannt.

Es wurde erfindungsgemäßes braunes Leder L3.4 erhalten, das über hervorragende Wasch-, Schweiß-, Reib- und Migrationsechtheit und hervorragende Fülle verfügt.

### III.5 Herstellung von erfindungsgemäßem Leder L3.5

a) Ein Lederstück von 100 Gewichtsteilen eines mit Glutardialdehyd gegerbten Rindsleders der Falzstärke 1,1 mm wurde in einem mit 200 Gewichtsteilen Wasser gefüllten Wackerfass 20 Minuten bei 30°C gewaschen. Danach wurde mit 150 Gewichtsteilen Wasser, 2 Gewichtsteilen eines Naphthalinsulfonsäure-Formaldehyd-Kondensationsprodukts, hergestellt nach US 5,186,846, Beispiel "Dispergiermittel 1", und 3 Gewichtsteilen eines Farbstoffes 5 min gewalkt.

Der Farbstoff wurde folgendermaßen hergestellt:
Schritt 1) 1 mol MSP (3-(2-Sulfatoethylsulfonyl)-6-aminobenzolsulfonsäure) wurde im Eiswasser suspendiert und bei pH-Wert ≤1 und einer Temperatur von 0 bis 5°C mit überschüssigem Natriumnitrit und Salzsäure diazotiert. 1 mol Gammasäure wurde in 25 Gew.-% Natronlauge gelöst und dann durch Zugabe von 21 Gew.-% Salzsäure der pH-Wert unter 1 gestellt. Die Gammasäure fiel aus. Zu dieser Suspension wurde die diazotierte MSP eingestürzt. Die Reaktionsmischung wurde bei pH-Wert ≤1 eine Stunde nachgerührt, bis kein diazotiertes MSP bzw. freie Gammasäure mehr nachweisbar war. Danach wurde der pH-Wert durch Zugabe von 15 Gew.-% Natriumcarbonatlösung auf 3 bis 8 angehoben.
Schritt 2) 1 mol 4,6-Dinitro-2-aminophenol wurde mit überschüssigem Natriumnitrit und Salzsäure bei pH-Wert ≤1 und einer Temperatur von 0 bis 5°C diazotiert und anschließend zu der in Schritt 1) erhaltenen Reaktionsmischung gegeben. Der pH-Wert wurde durch Zugabe von 10 Gew.-% Natronlauge zwischen 3 und 8 gehalten. Nach beendeter Umsetzung wurde 0,20-0,30 mol Chrom(III)-sulfat zu der Reaktionsmischung gegeben und 1 Stunde nachgerührt. Der Farbstoff wurde durch Zugabe von NaCl ausgesalzen und abgesaugt. Anschließend wurden 10 Gewichtsteile erfindungsgemäße Dispersion D2 zu der Flotte zugegeben und 5 Minuten gewalkt. Anschließend wurden 15 Gewichtsteile erfindungsgemäße Verbindung 1.4 zugegeben und 60 min bei pH-Wert 4,4 und 30°C behandelt. Durch portionsweise Zugabe von 100 Gewichtsteilen 15 Gew.-% Sodalösung wurde bei 40°C der pH-Wert zwischen 10,0 und 10,2 zur Fixierung der Verbindungen 120 Minuten lang unter Walken gehalten. Anschließend folgte sechsmal eine je 10 minütige Waschoperation in 200 Gewichtsteilen Wasser bei 40°C. Durch Zugabe von 200 Gewichtsteilen Wasser und 0,7 Gewichtsteilen Ameisensäure wurde ein pH-Wert von 4,7 eingestellt.

Das gefärbte, nachgegerbte und gefettete Leder wurde noch mit 15°C kalten Wasser 10 Minuten gespült und anschließend ausgereckt, über Nacht bei Raumtemperatur hängegetrocknet, konditioniert, gestollt und gespannt.

Es wurde erfindungsgemäßes Leder L3.5 erhalten, das über hervorragende Farbtiefe, Wasch-, Schweiß-, Reib- und Migrationsechtheit, hervorragende Weichheit und Fülle und sehr gute Fogging-Werte verfügt.

## Patentansprüche

1. Verfahren zur Herstellung von Leder, **dadurch gekennzeichnet, dass** man vorbehandelte Tierhäute mit wenigstens einer Verbindung kontaktiert, die wenigstens eine Gruppe der Formel I aufweist, 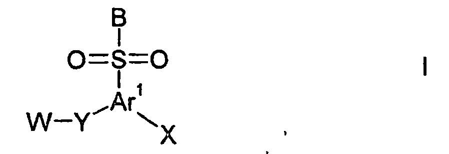 wobei die Variablen wie folgt definiert sind;
Ar¹ ein aromatisches oder heteroaromatisches System,
X ein elektronenziehender Rest oder Wasserstoff,
Y eine bivalente funktionelle Gruppe oder eine Einfachbindung oder eine C₂-C₂₀-Alkylengruppe, bei der bis zu 3 nicht-benachbarte CH₂-Gruppen durch Sauerstoff oder -NR³- ersetzt sein können,
W ein Rest, der von einer Verbindung abgeleitet ist, die auf Leder hydrophobierend, fettend oder gerbend wirkt,
B gewählt aus -C(R¹)=CH₂ und CHR¹-CH₂-Q, wobei
R¹ gewählt wird aus Wasserstoff, C₁-C₁₀-Alkyl und Phenyl,
R³ gleich oder verschieden, aus Phenyl, Wasserstoff oder C₁-C₁₀-Alkyl und
Q aus unter wässrig-alkalischen Bedingungen abspaltbaren Gruppen.

2. Verfahren nach Anspruch 1, **dadurch gekennzeichnet, dass** W gewählt wird aus mono-, di- oder polyfunktionalen Polysiloxanresten, C₈-C₅₀₀-Alkylresten, C₈-C₆₀-Alkylenresten, mehrfach fluorierten C₁-C₂₀-Alkylresten, Poly(meth)-acrylsäuregruppen, Phenolsulfonsäure-Hamstoff-Formaldehyd-Kondensaten.

3. Verfahren nach Anspruch 1 oder 2, **dadurch gekennzeichnet, dass** Ar¹ gewählt wird aus Benzolsystemen, Naphthalinsystemen, Anthracensystemen, Phenanthrensystemen, Pyridinsystemen, Chinolinsystemen, Isochinolinsystemen, Acridinsystemen, Pyrazinsystemen, Pyridazinsystemen, Pyrimidinsystemen, Chinazolinsystemen, Chinoxalinsystemen und Triazinsystemen.

4. Verfahren nach einem der Ansprüche 1 bis 3, **dadurch gekennzeichnet, dass** man es bei einem pH-Wert im Bereich von 7 bis 10 durchführt.

5. Verfahren nach einem der Ansprüche 1 bis 4, **dadurch gekennzeichnet, dass** Q für O-SO₃M, OSO₂-Ar² oder O-CO-R² steht, wobei
M für Wasserstoff oder Alkalimetall oder Ammonium steht,
Ar² für Phenyl steht, unsubstituiert oder ein- oder mehrfach substituiert mit Halogen, NO₂, C₁-C₁₀-Alkyl oder SO₃M.
und R² für Wasserstoff, C₁-C₁₀-Alkyl oder Phenyl.

6. Verfahren nach einem der Ansprüche 1 bis 5, **dadurch gekennzeichnet, dass** mindestens ein elektronenziehender Rest X gewählt wird aus SO₃M.

7. Verfahren nach einem der Ansprüche 1 bis 6, **dadurch gekennzeichnet, dass** Y gewählt wird aus einer funktionellen Gruppe Y.1 bis Y.9 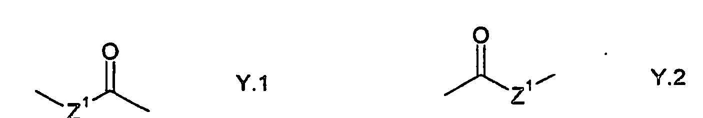 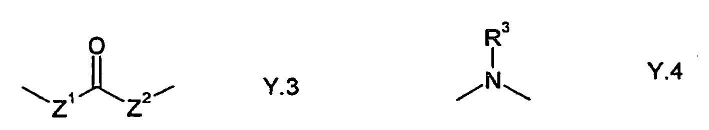 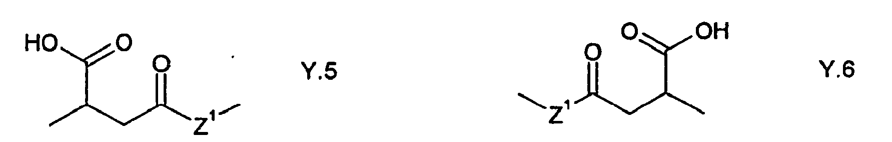 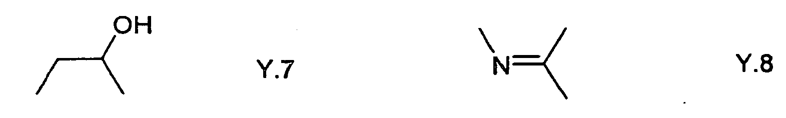 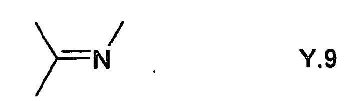 wobei Z¹ und Z² gleich oder verschieden sein können und gewählt aus Sauerstoff und N-R³.

8. Leder, hergestellt nach einem Verfahren nach einem der Ansprüche 1 bis 7.

9. Verwendung von Leder nach Anspruch 8 zur Herstellung von Bekleidungsstücken, Möbeln, Möbelteilen oder Autoteilen.

10. Verbindungen, enthaltend wenigstens eine Gruppe der allgemeinen Formel I 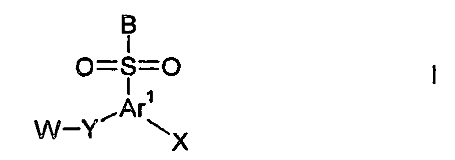 wobei die Variablen wie folgt definiert sind:
Ar¹ ein aromatisches oder heteroaromatisches System,
X ein elektronenziehender Rest oder Wasserstoff,
Y eine bivalente funktionelle Gruppe oder eine Einfachbindung oder eine C₂-C₂₀-Alkylengruppe, bei der bis zu 3 nicht-benachbarte CH₂-Gruppen durch Sauerstoff oder -NR³- ersetzt sein können,
W ein Rest, der von einer Verbindung abgeleitet ist, die auf Leder hydrophobierend, fettend oder gerbend wirkt,
B gewählt aus -C(R¹)=CH₂ und CHR¹-CH₂-Q, wobei
R¹ gewählt wird aus Wasserstoff, C₁-C₁₀-Alkyl und Phenyl,
R3 gleich oder verschieden, aus Phenyl, Wasserstoff oder C1-C10-Alkyl und
Q aus unter wässrig-alkalischen Bedingungen abspaltbaren Gruppen.

11. Verbindungen nach Anspruch 10, **dadurch gekennzeichnet, dass** W gewählt wird aus mono-, di- oder polyvalenten Polysiloxanresten, C₈-C₅₀₀-Alkylresten, C₈-C₆₀-Alkylenresten, mehrfach fluorierten C₁-C₂₀-Alkylresten, Poly(meth)acrylsäuren, Phenolsulfonsäure-Harnstoff-Formaldehyd-Kondensate.

12. Verbindungen nach Anspruch 10 oder 11, **dadurch gekennzeichnet, dass** Ar¹ gewählt wird aus Benzolsystemen, Naphthalinsystemen, Anthracensystemen, Phenanthrensystemen, Pyridinsystemen, Chinolinsystemen, lsochinolinsystemen, Acridinsystemen, Pyrazinsystemen, Pyridazinsystemen, Pyrimidinsystemen, Chinazolinsystemen, Chinoxalinsystemen und Triazinsystemen.

13. Verbindungen nach einem der Ansprüche 10 bis 12, **dadurch gekennzeichnet, dass** Q für O-SO₃M, OSO₂-Ar² oder O-CO-R² steht, wobei
M für Wasserstoff oder Alkalimetall oder Ammonium steht,
Ar² für Phenyl steht, unsubstituiert oder ein- oder mehrfach substituiert mit Halogen, NO₂, C₁-C₁₀-Alkyl oder SO₃M.
und R² für Wasserstoff, C₁-C₁₀-Alkyl oder Phenyl.

14. Verbindungen nach einem der Ansprüche 10 bis 13, **dadurch gekennzeichnet, dass** mindestens ein elektronenziehender Rest X gewählt wird aus SO₃M.

15. Verbindungen nach einem der Ansprüche 10 bis 14, **dadurch gekennzeichnet, dass** Y gewählt wird aus einer funktionellen Gruppe Y.1 bis Y.9 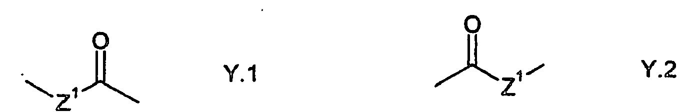 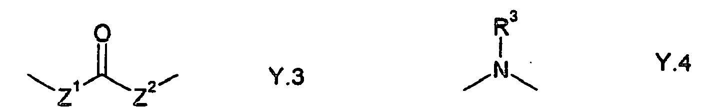 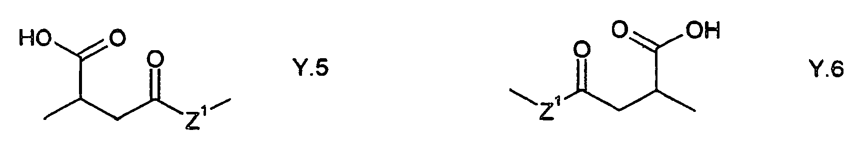 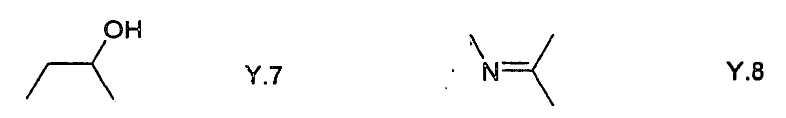 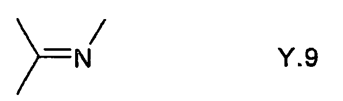 wobei Z¹ und Z² gleich oder verschieden sein können und gewählt aus Sauerstoff und N-R³.

16. Verfahren zur Herstellung von Verbindungen nach mindestens einem der Ansprüche 10 bis 15, **dadurch gekennzeichnet, dass** man mindestens eine Verbindung II, III, V a bis V c oder VI 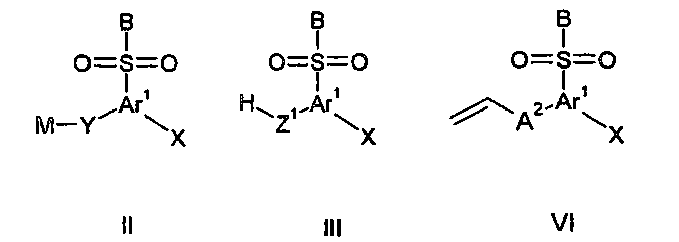 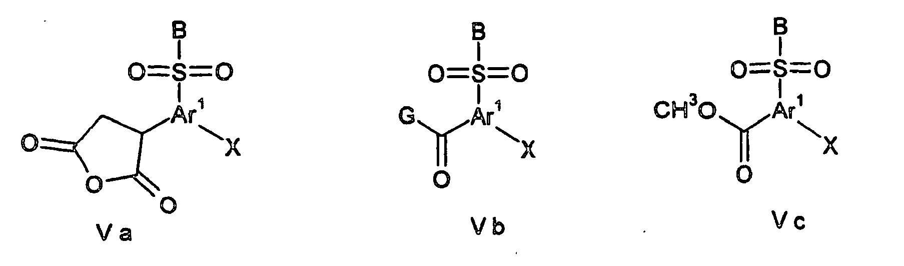 mit mindestens einem Derivat von W, gegebenenfalls in Gegenwart eines Katalysators, umsetzt, wobei
G für Halogen steht und
A² für eine Einfachbindung oder C¹-C¹⁸-Alkylen, in dem bis zu 3 nicht-benachbarte CH₂-Gruppen durch Sauerstoff oder NR³ ersetzt sein können.

17. Wässrige Dispersion, enthaltend mindestens eine Verbindung nach Anspruch 10 bis 15.

## Claims

1. A process for the production of leather, wherein pretreated animal hides are brought into contact with at least one compound which has at least one group of the formula I 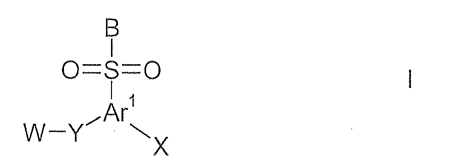 where the variables are defined as follows:
Ar¹ an aromatic or heteroaromatic system,
X an electron-attracting radical or hydrogen,
Y a bivalent functional group or a single bond or a C₂-C₂₀-alkylene group in which up to 3 non-neighboring CH₂ groups may be replaced by oxygen or -NR³-,
W a radical which is derived from a compound which has a water-repellent, fatliquoring or tanning effect on leather,
B selected from -C(R¹)=CH₂ and CHR¹-CH₂-Q, where
R¹ selected from hydrogen, C₁-C₁₀-alkyl and phenyl,
R³ identical or different, from phenyl, hydrogen or C₁-C₁₀-alkyl, and
Q from groups which can be eliminated under aqueous alkaline conditions.

2. The process according to claim 1, wherein W is selected from mono-, di- or polyfunctional polysiloxane radicals, C₈-C₅₀₀-alkyl radicals, C₈-C₆₀-alkylene radicals, polyfluorinated C₁-C₂₀-alkyl radicals, poly(meth)acrylic acid groups, phenolsulfonic acid/urea/formaldehyde condensates.

3. The process according to claim 1 or 2, wherein Ar¹ is selected from benzene systems, naphthalene systems, anthracene systems, phenanthrene systems, pyridine systems, quinoline systems, isoquinoline systems, acridine systems, pyrazine systems, pyridazine systems, pyrimidine systems, quinazoline systems, quinoxaline systems and triazine systems.

4. The process according to any of claims 1 to 3, which is carried out at a pH in the range from 7 to 10.

5. The process according to any of claims 1 to 4, wherein Q is O-SO₃M, OSO₂-Ar² or O-CO-R², where
M is hydrogen or an alkali metal or ammonium,
Ar² is phenyl, unsubstituted or mono- or polysubstituted by halogen, NO₂, C₁-C₁₀-alkyl or SO₃M,
and R² is hydrogen, C₁-C₁₀-alkyl or phenyl.

6. The process according to any of claims 1 to 5, wherein at least one electron-attracting radical X is selected from SO₃M.

7. The process according to any of claims 1 to 6, wherein Y is selected from a functional group Y.1 to Y.9 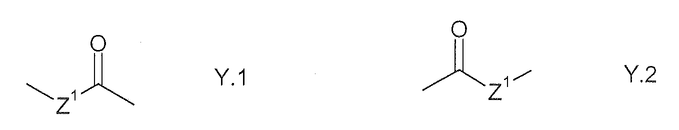 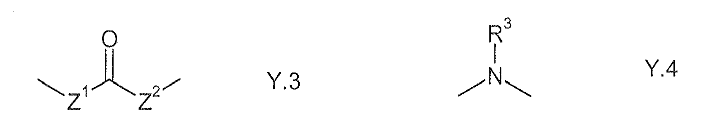 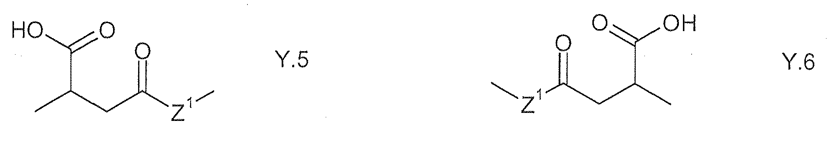 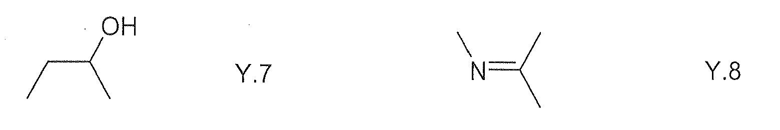 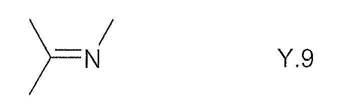 where Z¹ and Z² may be identical or different and are selected from oxygen and N-R³.

8. A leather produced by a process according to any of claims 1 to 7.

9. The use of the leather according to claim 8 for the production of articles of apparel, pieces of furniture, furniture parts or automotive parts.

10. A compound comprising at least one group of the general formula I 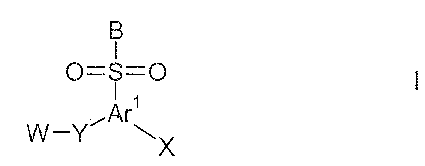 where the variables are defined as follows:
Ar¹ an aromatic or heteroaromatic system,
X an electron-attracting radical or hydrogen,
Y a bivalent functional group or a single bond or a C₂-C₂₀-alkylene group in which up to 3 non-neighboring CH₂ groups may be replaced by oxygen or -NR³- ,
W a radical which is derived from a compound which has a water-repellent, fatliquoring or tanning effect on leather,
B selected from -C(R¹)=CH₂ and CHR¹-CH₂-Q, where
R¹ selected from hydrogen, C₁-C₁₀-alkyl and phenyl,
R³ identical or different, from phenyl, hydrogen or C₁-C₁₀-alkyl, and
Q from groups which can be eliminated under aqueous alkaline conditions.

11. The compound according to claim 10, wherein W is selected from mono-, di- or polyvalent polysiloxane radicals, C₈-C₅₀₀-alkyl radicals, C₈-C₆₀-alkylene radicals, polyfluorinated C₁-C₂₀-alkyl radicals, poly(meth)acrylic acids, phenolsulfonic acid/urea/formaldehyde condensates.

12. The compound according to claim 10 or 11, wherein Ar¹ is selected from benzene systems, naphthalene systems, anthracene systems, phenanthrene systems, pyridine systems, quinoline systems, isoquinoline systems, acridine systems, pyrazine systems, pyridazine systems, pyrimidine systems, quinazoline systems, quinoxaline systems and triazine systems.

13. The compound according to any of claims 10 to 12, wherein Q is O-SO₃M, OSO₂-Ar² or O-CO-R², where
M is hydrogen or an alkali metal or ammonium,
Ar² is phenyl, unsubstituted or mono- or polysubstituted by halogen, NO₂, C₁-C₁₀-alkyl or SO₃M.
and R² is hydrogen, C₁-C₁₀-alkyl or phenyl.

14. The compound according to any of claims 10 to 13, wherein at least one electron-attracting radical X is selected from SO₃M.

15. The compound according to any of claims 10 to 14, wherein Y is selected from a functional group Y.1 to Y.9 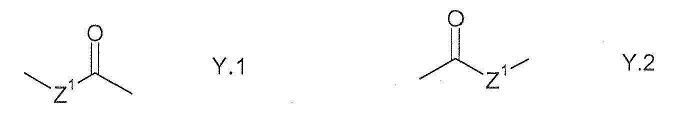 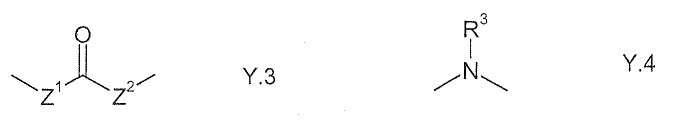 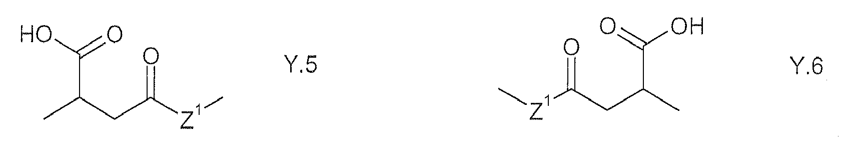 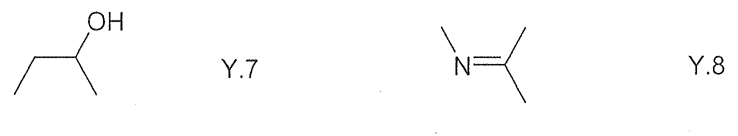 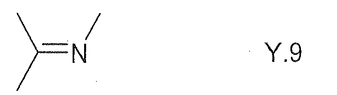 where Z¹ and Z² may be identical or different and are selected from oxygen and N-R³.

16. A process for the preparation of compounds according to at least one of claims 10 to 15, wherein at least one compound II, III, V a to V c or VI 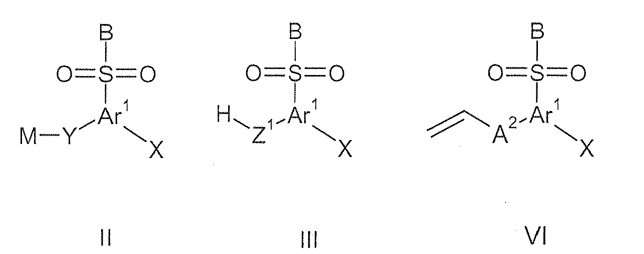 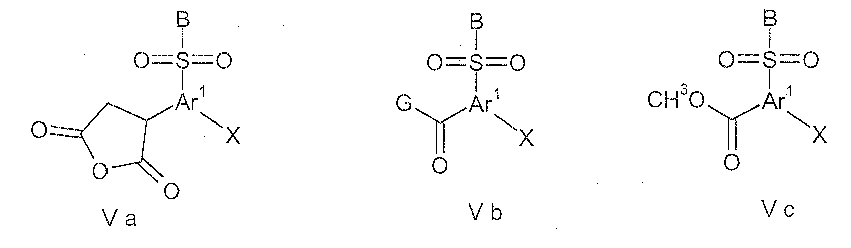 is reacted with at least one derivative of W, if appropriate in the presence of a catalyst, where
G is halogen and
A² is a single bond or C₁-C₁₈-alkylene in which up to 3 non-neighboring CH₂ groups may be replaced by oxygen or NR³.

17. An aqueous dispersion comprising at least one compound according to any of claims 10 to 15.

## Revendications

1. Procédé de fabrication de cuir, **caractérisé en ce que** des peaux d'animaux prétraitées sont mises en contact avec au moins un composé qui comporte au moins un groupement de formule I, 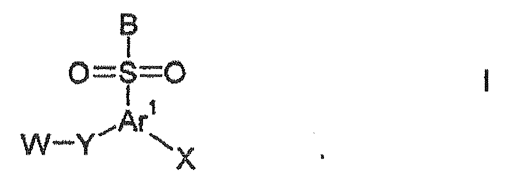 dans laquelle les variables sont définies tel que suit :
Ar¹ un système aromatique ou hétéroaromatique
X un radical attracteur d'électrons ou l'hydrogène
Y un groupement fonctionnel bivalent ou une simple liaison ou un groupement alkylène en C₂ à C₂₀ dont 3 groupements CH₂ non voisins au plus peuvent être remplacés par un oxygène ou -NR³-,
W un radical qui dérive d'un composé qui a une action hydrophobante, lubrifiante ou tannante sur le cuir,
B choisi parmi -C(R¹)=CH₂ et CHR¹-CH₂-Q, avec
R¹ choisi parmi l'hydrogène, un alkyle en C₁ à C₁₀ et un phényle
R³ identiques ou différents, choisis parmi un phényle, l'hydrogène ou un alkyle en C₁ à C₁₀ et
Q choisi parmi les groupements clivables en conditions aqueuses alcalines.

2. Procédé selon la revendication 1, **caractérisé en ce que** W est choisi parmi les radicaux polysiloxane mono-, di- ou polyfonctionnels, les radicaux alkyle en C₈ à Csoo, les radicaux alkylène en C₈ à C₆₀, les radicaux alkyle en C₁ à C₂₀ plusieurs fois fluorés, les groupements acide poly(méth)acrylique, les condensats acide phénolsulfonique-urée-formaldéhyde.

3. Procédé selon la revendication 1 ou 2, **caractérisé en ce que** Ar¹ est choisi parmi les systèmes benzène, les systèmes naphtaline, les systèmes anthracène, les systèmes phénanthrène, les systèmes pyridine, les systèmes quinoléine, les systèmes isoquinoléine, les systèmes acridine, les systèmes pyrazine, les systèmes pyridazine, les systèmes pyrimidine, les systèmes quinazoline, les systèmes quinoxaline et les systèmes triazine.

4. Procédé selon l'une quelconque des revendications 1 à 3, **caractérisé en ce qu'**il est réalisé à un pH de 7 à 10.

5. Procédé selon l'une quelconque des revendications 1 à 4, **caractérisé en ce que** Q représente O-SO₃M, OSO₂-Ar² ou O-CO-R², avec
M représentant l'hydrogène ou un métal alcalin ou l'ammonium
Ar² représentant un phényle non substitué ou substitué une ou plusieurs fois avec un halogène, NO₂, un alkyle en C₁ à C₁₀ ou SO₃M,
et R² représentant l'hydrogène, un alkyle en C₁ à C₁₀ ou un phényle.

6. Procédé selon l'une quelconque des revendications 1 à 5, **caractérisé en ce qu'**au moins un radical attracteur d'électrons X est choisi parmi SO₃M.

7. Procédé selon l'une quelconque des revendications 1 à 6, **caractérisé en ce que** Y est choisi parmi les groupements fonctionnels Y.1 à Y.9 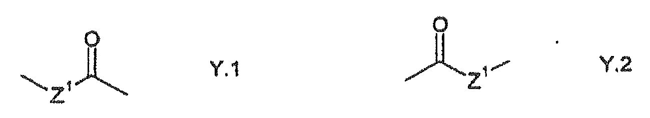 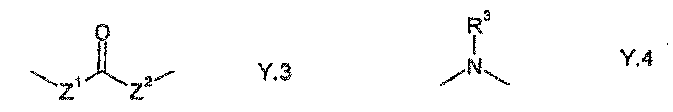 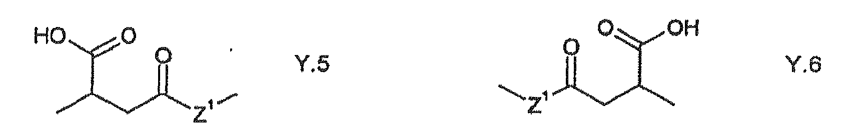 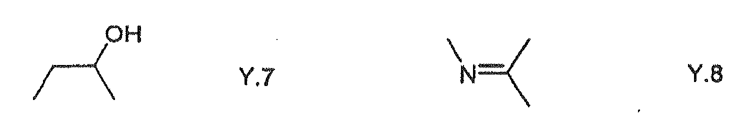 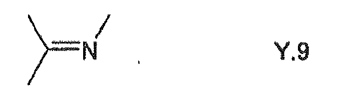 dans lesquels Z¹ et Z² peuvent être identiques ou différents et sont choisis parmi l'oxygène et N-R³.

8. Cuir, fabriqué par un procédé selon l'une quelconque des revendications 1 à 7.

9. Utilisation d'un cuir selon la revendication 8 pour la fabrication d'éléments de vêtements, de meubles, de parties de meubles ou de parties d'automobiles.

10. Composés, contenant au moins un groupement de formule générale I 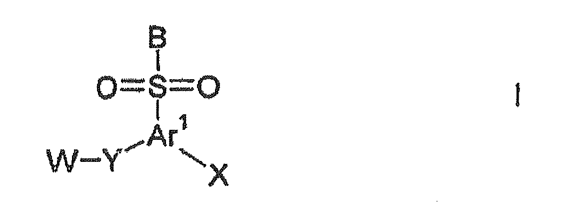 dans laquelle les variables sont définies tel que suit :
Ar¹ un système aromatique ou hétéroaromatique
X un radical attracteur d'électrons ou l'hydrogène
Y un groupement fonctionnel bivalent ou une simple liaison ou un groupement alkylène en C₂ à C₂₀ dont 3 groupements CH₂ non voisins au plus peuvent être remplacés par un oxygène ou -NR³-,
W un radical qui dérive d'un composé qui a une action hydrophobante, lubrifiante ou tannante sur le cuir,
B choisi parmi -C(R¹)=CH₂ et CHR¹-CH₂-Q, avec
R¹ choisi parmi l'hydrogène, un alkyle en C₁ à C₁₀ et un phényle
R³ identiques ou différents, choisis parmi un phényle, l'hydrogène ou un alkyle en C₁ à C₁₀ et
Q choisi parmi les groupements clivables en conditions aqueuses alcalines.

11. Composés selon la revendication 10, **caractérisés en ce que** W est choisi parmi les radicaux polysiloxane mono-, di- ou polyvalents, les radicaux alkyle en C₈ à C₅₀₀, les radicaux alkylène en C₈ à C₆₀, les radicaux alkyle en C₁ à C₂₀ plusieurs fois fluorés, les acides poly(méth)acryliques, les condensats acide phénolsulfonique-urée-formaldéhyde.

12. Composés selon la revendication 10 ou 11, **caractérisés en ce que** Ar¹ est choisi parmi les systèmes benzène, les systèmes naphtaline, les systèmes anthracène, les systèmes phénanthrène, les systèmes pyridine, les systèmes quinoléine, les systèmes isoquinoléine, les systèmes acridine, les systèmes pyrazine, les systèmes pyridazine, les systèmes pyrimidine, les systèmes quinazoline, les systèmes quinoxaline et les systèmes triazine.

13. Composés selon l'une quelconque des revendications 10 à 12, **caractérisés en ce que** Q représente O-SO₃M, OSO₂-Ar² ou O-CO-R², avec
M représentant l'hydrogène ou un métal alcalin ou l'ammonium
Ar² représentant un phényle non substitué ou substitué une ou plusieurs fois avec un halogène, NO₂, un alkyle en C₁ à C₁₀ ou SO₃M,
et R² représentant l'hydrogène, un alkyle en C₁ à C₁₀ ou un phényle.

14. Composés selon l'une quelconque des revendications 10 à 13, **caractérisés en ce qu'**au moins un radical attracteur d'électrons X est choisi parmi SO₃M.

15. Composés selon l'une quelconque des revendications 10 à 14, **caractérisés en ce que** Y est choisi parmi les groupements fonctionnels Y.1 à Y.9 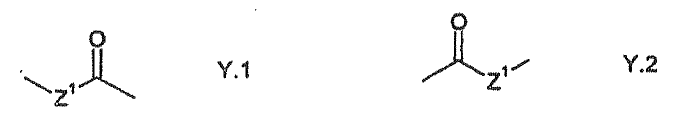 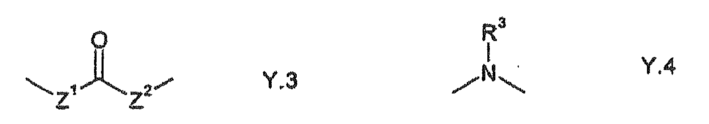 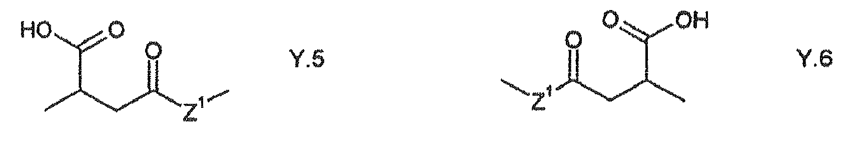 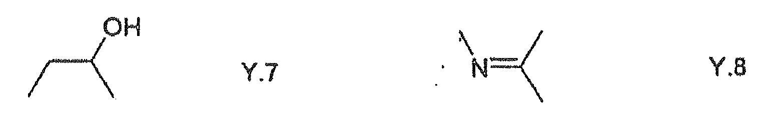 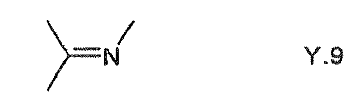 dans lesquels Z¹ et Z² peuvent être identiques ou différents et sont choisis parmi l'oxygène et N-R³.

16. Procédé de fabrication de composés selon au moins l'une des revendications 10 à 15, **caractérisé en ce qu'**au moins un composé II, III, Va à Vc ou VI 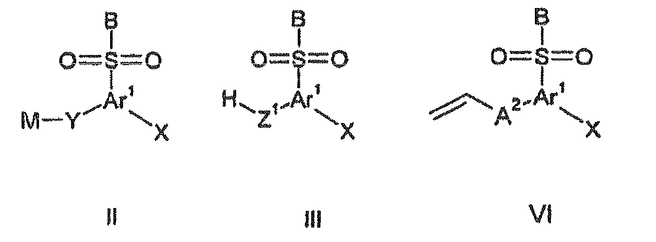 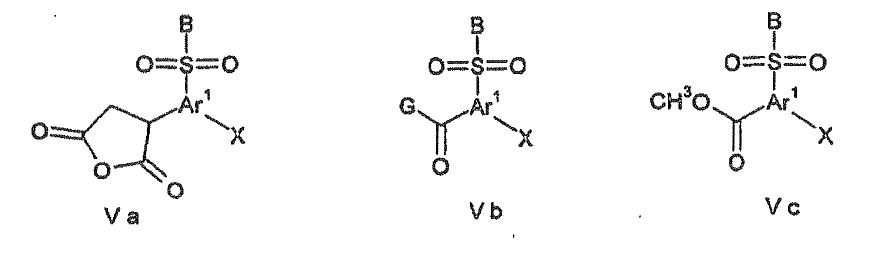 est mis en réaction avec au moins un dérivé de W, éventuellement en présence d'un catalyseur,
G représentant un halogène et
A² représentant une simple liaison ou un alkylène en C₁ à C₁₈ dont 3 groupements CH₂ non voisins au plus peuvent être remplacés par un oxygène ou un NR³.

17. Dispersion aqueuse, contenant au moins un composé selon les revendications 10 à 15.
